# FASCICULE DE BREVET EUROPEEN

(11) **EP 2 880 045 B1**
(45) Date de publication et mention de la délivrance du brevet: **20.07.2016**
(21) Numéro de dépôt: 13756639.4
(22) Date de dépôt: 31.07.2013
(51) Int. Cl.: C07H 15/26, A61K 49/00, C12Q 1/34

(54) **SUBSTRAT DE GLYCOSIDASE FLUOROGÈNE ET PROCÉDÉ DE DÉTECTION ASSOCIÉ**
FLUOROGENES GLYCOSIDASE-SUBSTRAT UND ZUGEHÖRIGES DETEKTIONSVERFAHREN
FLUOROGENIC GLYCOSIDASE SUBSTRATE AND ASSOCIATED DETECTION PROCESS

(30) Priorité: 02.08.2012 FR 1257515
(43) Date de publication de la demande: 10.06.2015
(73) Titulaire: Ecole Normale Supérieure de Lyon, 69342 Lyon Cedex 07 (FR); Université Claude Bernard Lyon I, 69622 Villeurbanne Cedex (FR); Centre National de la Recherche Scientifique (CNRS), 75794 Paris Cedex 16 (FR)
(72) Inventeur: HASSERODT, Jens, F-69110 SAINTE FOY LES LYON (FR)
(74) Mandataire: Sarlin, Laure V.
(86) Numéro de dépôt international: PCT/FR2013/051855
(87) Numéro de publication internationale: WO 2014/020285

(56) Documents cités:
- WO-A1-2010/146164
- WO-A2-2008/145830
- FENGTIAN XUE AND CHRISTOPHER T. SETO: "Kinetic delay of cyclization/elimination-coupled enzyme assays: Analysis and solution", BIOORGANIC & MEDICINAL CHEMISTRY LETTERS, vol. 21, no. 3, 2011, pages 1069-1071, XP055042989, ISSN: 0960-894X, DOI: 10.1016/j.bmcl.2010.09.104
- FRANCISCUS M. H. DE GROOT ET AL: "Elongated multiple electronic cascade and cyclization spacer systems in activatible anticancer prodrugs for enhanced drug release", JOURNAL OF ORGANIC CHEMISTRY, vol. 66, 2001, pages 8815-8830, XP002212035, ISSN: 0022-3263, DOI: 10.1021/JO0158884

## Description

L'invention concerne le domaine technique des sondes pour détecter une activité enzymatique de type glycosidase. En particulier, l'invention concerne de nouveaux substrats fluorogènes pour détecter la présence d'une glycosidase catalytiquement active et un procédé de détection utilisant de tels substrats.

Dans l'analyse d'un échantillon biologique ou chimique, la détection d'une activité glycosidase peut être très utile (Boonacker E. et Van Noorden C.J.F. (2001) J. Histochem. Cytochem. 49, 1473-1486 ; Perry, J. D., James, A. L., Morris, K. A., Oliver, M., Chilvers, K. F., Reed, R. H., & Gould, F. K. (2006). Evaluation of nove! fluorogenic substrates for the detection of glycosidases in Escherichia coli and enterococci. Journal of Applied Microbiology, 101(5), 977-985 ; Orenga, S., James, A. L., Manafi, M., Perry, J. D., & Pincus, D. H. (2009). Enzymatic substrates in microbiology. Journal of Microbiological Methods, 79(2), 139-155). Les organismes entiers, les cellules ou les extraits cellulaires, les liquides biologiques ou les mélanges chimiques sont des exemples d'échantillons biologiques ou chimiques dans lesquels une activité glycosidase peut être détectée. Les glycosidases sont une vaste famille d'enzymes qui inclut de nombreux biomarqueurs de diverses pathologies. Elles sont impliquées aussi dans de nombreux processus cellulaires bénins et font donc l'objet d'études innombrables de la part des biologistes cellulaires. Ainsi, leur détection peut donner des informations concernant un état métabolique ou morbide particulier, par exemple.

De ce fait, une sonde capable de détecter une activité glycosidase est très utile. La détection de cette activité par capture de lumière de fluorescence est un procédé bien plus sensible que la collecte du reste de lumière blanche lors d'une simple absorption par la sonde, c'est-à-dire que le seuil de détection est bien plus bas. La détection d'une émission de fluorescence est très aisée à mettre en oeuvre, de sorte que les sondes fluorescentes sont des outils très attractifs pour les sciences de la vie. Par exemple, la classe des fluorophores conduisant à un transfert de proton intramoléculaire dans un état excité, nommés ESIPT (ESIPT de l'anglais « Excited State Intramolecular Proton Transfer »), est décrite, notamment, dans a) Ormson, S.M., et al. Progress in Reaction Kinetics (1994) 19, 45-91 ; b) Legourrierec, D., et al. Progress in Reaction Kinetics (1994), 19, 211-275; et c) Zhao, J., Ji, S., Chen, Y., Guo, H., & Yang, P. (2012). Excited state intramolecular proton transfer (ESIPT): from principal photophysics to the development of new chromophores and applications in fluorescent molecular probes and luminescent materials. Physical chemistry chemica/ physics, 14(25), 8803). La première interprétation de la fluorescence élevée trouvée dans certains composés phénoliques comme étant un phénomène ESIPT peut être attribuée à Weller (pour le salicylate de méthyle : Weller, A. (1961), Fast Reactions of Excited Molecules. Progress in Reaction Kinetics and Mechanism 1, 187), et à Heller et Williams (pour les hydroxyphenylbenzoxazoles : Heller A., et Williams, D.L., J. Phys. Chem. (1970)74, 4473-4480),

La classe des fluorophores ESIPT est particulièrement attractive pour le chercheur dans les sciences de la vie, du fait de ses propriétés exceptionnelles comparées aux fluorophores conventionnels. Les propriétés exceptionnelles des fluorophores ESIPT sont :
(a) un grand déplacement de Stokes dépassant souvent 130 nm et capable d'atteindre des valeurs de 250 nm qui permet des choix instrumentaux maximisant la sensibilité de détection ;
(b) une excellente résistance au photoblanchiment avec des taux qui peuvent être d'ordres de grandeur supérieurs à ceux de fluorophores modèles comme la fluorescéine;
(c) la possibilité de concevoir des fluorophores qui émettent une fluorescence brillante à l'état solide, une propriété rare parmi tous les fluorophores connus. Cette dernière performance permet la production d'un signal de haute intensité au site de radiation de la sonde, avec une dilution minimale causée par la diffusion ; et enfin,
(d) la possibilité de concevoir des fluorophores ESIPT qui émettent dans le rouge ou le proche infrarouge (600 à 850 nm) où la transparence des tissus est la plus élevée ; la sonde correspondante serait alors particulièrement appropriée pour l'imagerie chez l'animal vivant.

La majorité de ces propriétés, si un tel fluorophore était intégré dans une sonde, constituerait un apport important par rapport aux propriétés des sondes commercialisées conventionnelles qui sont entravées dans leurs performances par de petits déplacements de Stokes et un photoblanchiment moyen à élever, et qui conduisent sans exception à des fluorophores à l'état de solution.

Dans le cadre de l'invention, l'inventeur s'est intéressé au choix judicieux d'un motif clivable par une enzyme glycosidase qui confèrerait à la sonde complète, incorporant un fluorophore ESIPT comme décrit ci-dessus, une hydrosolubilité totale, condition préalable pour atteindre les sites et les tissus d'intérêt. Une telle sonde permettrait une augmentation significative de la sensibilité de détection, qui, de ce fait, permettrait une réduction de la dose et pourrait ainsi, notamment, être adaptée à une application en imagerie *in vivo,* tout en réduisant les problèmes de toxicité. Le niveau de sensibilité est étroitement lié (i) au taux de photoblanchiment, (ii) au degré d'accumulation du signal fluorescent sur son site de production (et donc au taux de diffusion depuis ce site, et à la question de savoir si le fluorophore précipite ou non) (iii) au mode éteint/allumé réel dans lequel la sonde fonctionne (absence de bruit de fond qui serait dû à une fluorescence de la sonde non transformée), et (iv) au degré de superposition du spectre d'excitation et du spectre d'émission (leur séparation au niveau de la ligne de base étant la configuration la plus favorable ; voir point (a) ci-dessus). Le point (iv) revêt une importance toute particulière, car la séparation complète au niveau de la ligne de base offre l'opportunité d'un choix de filtres très large pour la source lumineuse (pour exciter la molécule à toutes les longueurs d'ondes possibles), mais de manière encore plus importante, pour le détecteur (pour récolter des photons provenant de toutes les longueurs d'ondes émises par le fluorophore). Le point (iv) minimise aussi la perturbation du processus de détection par l'autofluorescence tissulaire (caractérisée par un faible déplacement de Stokes des fluorophores naturels), un problème récurrent rencontré avec les fluorophores établis, qui présentent eux aussi un faible déplacement de Stokes.

Ces dernières années, il y a eu un intérêt croissant dans la conception de substrats d'enzymes à trois composants déclencheur/espaceur/fluorophore en utilisant des espaceurs à auto-immolation comme agent de liaison. Parmi la classe importante des fluorophores ESIPT, la dichloro-HPQ (6-chloro-2-(5-chloro-2-hydroxyphenyl)-4(3*H*)-Quinazolinone ; numéro CAS : 28683-92-3) est particulièrement intéressante, étant donnée qu'elle est parfaitement insoluble dans les milieux aqueux/physiologiques, tout en étant fortement fluorescente à l'état solide et seulement à l'état solide. Néanmoins, il est très difficile d'utiliser la dichloro-HPQ dans la conception d'une sonde moléculaire qui renseigne sur l'activité d'une glycosidase. D'ailleurs, les principales activités pour lesquelles une sonde à base de HPQ a déjà été conçue (et commercialisée) sont celles de phosphatases, du fait de l'impossibilité de créer une sonde à base de HPQ stable avec un hydroxyle phénolique glycosylé car le produit résultant est enclin à une hydrolyse spontanée rapide qui, bien entendu, libère la dichloro-HPQ insoluble libre et produit ainsi un signal fluorescent erroné ("signal de fond"). Il convient également de noter que la commercialisation par Molecular Probes de tels composés glycosylés (ELF 97 substrat glucuronidase (no. E6587) et ELF 97 substrat chitinase/N-acetylglucosaminidase (no. E22011) a été interrompue en 2008 et leur étude par certains chercheurs est restée sans suite (a) Chen, K.-C, Wu, C.-H., Chang, C.-Y., Lu, W.-C., Tseng, Q., Prijovich, Z. M., Schechinger, W., et al. (2008). Directed Evolution of a Lysosomal Enzyme with Enhanced Activity at Neutral pH by Mammalian Cell-Surface Display. Chemistry Biology, 15(12), 1277-1286 ; b) Štrojsová, A., & Dyhrman, S. T. (2008). Cell-specific β-N-acetylglucosaminidase activity in cultures and field populations of eukaryotic marine phytoplankton. FEMS Microbiology Ecology, 64(3), 351-361). La raison est l'instabilité à l'hydrolyse intrinsèque des glycosides phénoliques, et en particulier de ceux qui sont construits à partir des phénols appauvris en électrons, telle que la dichloro-HPQ, En effet, il est connu que n'importe quel glycoside à base d'un nitrophénol (qui est un phénol aussi appauvri en électrons que la dichloro-HPQ) s'hydrolyse spontanément à pH physiologique. Il est également connu que ce problème de stabilité s'aggrave sévèrement à des pH plus acides (par exemple à un pH de 6,5), par comparaison au pH physiologique (pH 7,4).

Des solutions conduisant à une absence plus marquée, voire complète, de dégradations spontanées des sondes et donc de production de signaux erronés, une condition préalable fondamentale pour leur utilisation dans des applications *in vitro* et *in vivo,* ont été proposées à travers la construction d'une sonde à trois composantes. Celles-ci comprennent un espaceur para-hydroxy- ou para-amino-benzyle greffé sur le fluorophore phénolique (WO2008145830). Bien que de tels espaceurs para-hydroxybenzyle aient déjà été utilisés dans la conception de pro-médicaments depuis le début des années 1980 (Wakselman, M. New Journal of Chemistry (1983), 7, 439-447), ils possèdent un inconvénient majeur reconnu : l'utilisation d'espaceurs para-hydroxybenzyle dans des substrats d'enzymes artificiels conduit à l'alkylation permanente de la protéine, souvent à proximité ou à l'intérieur du site catalytique. Cette propriété négative a, par la suite, été utilisée de manière avantageuse dans de nombreux articles qui ont proposé des substrats inactivant les enzymes, c'est-à-dire conduisant à une enzyme qui n'est plus capable de convertir encore les molécules de substrat (Zhu, J.; Withers, S. G.; Reichardt, P. B.; Treadwell, E.; Clausen, T.P. Biochem. J. 1998, 332, 367 - 371), comme le montre le **Schéma 1** suivant.

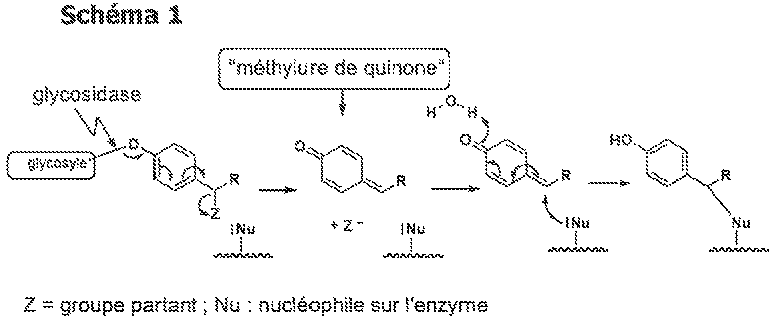

Les différents travaux sur le marquage enzymatique par des substrats d'enzyme à base d'ortho- ou para-hydroxybenzyle montrent que les méthylures de quinone sont des espèces très réactives qui alkylent les nucléophiles et qui risquent de modifier au hasard les propriétés moléculaires des biomacromolécules qui sont dans leur voisinage immédiat. L'activation ou les capacités catalytiques réduites de l'enzyme cible sont, bien entendu, très néfastes pour la sensibilité des expériences d'imagerie utilisant une telle sonde fluorogène, car l'amplification enzymatique est perdue. Mis à part l'inactivation directe de l'enzyme cible, l'alkylation aléatoire de la surface de cette protéine ou de toute autre protéine voisine présente également le risque d'une réponse immunitaire. Les deux cas génèrent une tolérance limitée de la part de l'organisme respectif et donc une toxicité élevée pour celui-ci (Grinda M. et al. ChemMedChem 2011, 6,2137 - 2141)

Dans ce contexte, la demanderesse s'est proposée d'offrir d'autres espaceurs 1) qui permettraient de créer une sonde stable incorporant un fluorophore ESIPT, avec ainsi une minimisation de la fluorescence de fond de la sonde non transformée, et 2) qui ne présenteraient pas les mêmes risques en termes de toxicité que l'espaceur hydroxybenzyle décrit dans l'état de la technique.

L'objectif de l'invention est de proposer de nouveaux substrats de glycosidase qui soient stables en milieu aqueux et qui restent non fluorescents ou faiblement fluorescents à une longueur d'onde bien différente de celle à laquelle le fluorophore libéré est lui fluorescent, mais qui réagissent avec des glycosidases pour produire une petite molécule fluorescente correspondant à un fluorophore ESIPT, comme la dichloro-HPQ. Selon l'invention, il est envisagé de proposer un substrat de glycosidase ayant les propriétés suivantes :
- possibilité d'obtenir une spécificité pour une glycosidase particulière, en fonction du choix du groupement glycosyl présent sur la sonde,
- absence de fluorescence de fond du fait de l'absence de dégradation spontanée de la sonde,
- bonne cinétique de transformation sous l'action de la glycosidase cible,
- biocompatibilité supérieure aux sondes utilisant des espaceurs hydroxybenzyle dont la toxicité est reconnue.

Plus précisément, l'invention concerne des composés de formule (I) : 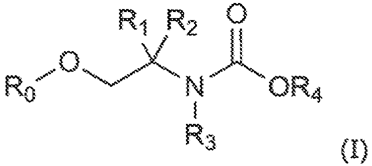 dans laquelle R₀, R₁, R₂, R₃ et R₄ sont tels que définis à la revendication 1,

sous la forme d'un mélange d'isomères optiques selon toutes proportions, ou sous une forme enrichie en un isomère optique.

Les composés selon l'invention, en fonction du groupe glycosyle sélectionné, agissent comme une sonde moléculaire capable de révéler la présence d'une activité enzymatique glycosidase spécifique par détection de fluorescence. La liaison R₀-O est susceptible d'être clivée, par hydrolyse, en présence d'une enzyme glycosidase agissant en tant que catalyseur de la réaction de clivage,

Plus spécifiquement, la sonde est invisible avant de rencontrer l'enzyme glycosidase ciblée, (c'est-à-dire "sonde furtive"), mais quand elle est modifiée chimiquement par ladite enzyme, elle se fragmente *via* une réaction en cascade pour produire une fluorescence intense. La sonde est composée de trois composants moléculaires i) un espaceur -OCH₂C(R₁R₂)N(R₃)C(O)- intelligent qui porte, à une extrémité, ii) un groupe glycosyle jouant le rôle de substrat pour l'enzyme cible et, à l'autre extrémité, iii) un groupe OR₄ qui, quand il est libéré sous sa forme hydroxylée par ladite fragmentation, appartient à la classe des fluorophores ESIPT.

Pour surmonter la cinétique de transformation défavorable qui est associée avec un espaceur hydroxyéthylcarbamate qui porte un groupe hydroxy primaire en position distale par rapport au groupement carbamate tertiaire, la présente invention propose une nouvelle famille d'espaceur du type hydroxy-amines qui sont substitués de telle manière qu'ils sont pré-organisés pour la cyclisation en une carbamate acyclique, Cette pré-organisation accélère le processus de transformation lors de l'activation par une enzyme.

Ce choix particulier d'espaceurs permet d'obtenir deux propriétés fondamentales pour la sonde moléculaire correspondante : (a) il l'a rend insensible à la dégradation spontanée et donc à la production d'un signal fluorescent faussement positif, et (b) il garantit une cinétique de fragmentation rapide lors de la transformation par l'enzyme cible pour une performance adaptée aux applications dans le domaine des sciences de la vie. Le groupe R₀ est capable d'être clivé du reste de la molécule par l'action de la glycosidase cible, ce qui conduit à un intermédiaire instable qui s'immole par une réaction de cyclisation/clivage spontanément et rapidement pour libérer un précipité fluorescent et ainsi produire un signal fluorescent.

La présente invention concerne donc les composés de formule (I), quelle que soit leur variante de mise en oeuvre décrite dans la présente demande de brevet, pour la détection *in vivo,* chez l'homme ou l'animal, d'une enzyme glycosidase.

Selon un autre aspect, l'invention concerne un procédé *in vitro* pour détecter la présence d'une enzyme glycosidase catalytiquement active, au moyen d'un composé de formule (I) selon l'invention. Plus précisément, l'invention concerne un procédé pour détecter la présence d'une enzyme glycosidase catalytiquement active comprenant les étapes de :
- mise en contact d'un échantillon suspecté de contenir ladite glycosidase avec un composé de formule (I) selon l'invention ;
- application de conditions appropriées pour permettre la formation d'un composé fluorescent, notamment sous la forme d'un précipité, par clivage de la liaison covalente entre -O-R₀, suivi d'un clivage de la liaison -C(O)-OR₄ suite à une cyclisation de L'espaceur -OCH₂C(R₁R₂)N(R₃)C(O)-, et
- analyse quantitative ou qualitative dudit précipité fluorescent.

Le précipité qui peut être obtenu à partir des composés de formule (I) selon l'invention, par clivage de la liaison covalence entre O et R₀, suivi d'un clivage de la liaison -C(O)-OR₄ suite à une cyclisation de l'espaceur - OCH₂C(R₁R₂)N(R₃)C(O)-, est fortement fluorescent, alors que le composé de formule (I) correspondant est peu fluorescent ou pas fluorescent du tout. Les composés selon l'invention qui sont des substrats d'enzyme glycosidase, se comportent comme des sondes fonctionnant selon le mode éteint/allumé.

En particulier, le procédé de détection selon l'invention peut être mis en oeuvre dans des conditions physiologiques, notamment dans un milieu aqueux tamponné à un pH de l'ordre de 7,4.

Dans un mode de réalisation de l'invention, l'analyse du composé ou précipité fluorescent comprend les étapes suivantes :
- exposition du précipité fluorescent à une source lumineuse capable de produire une lumière à une longueur d'onde d'absorption du précipité fluorescent ; et
- détection de la fluorescence résultante du précipité.

L'invention va être décrite de manière plus détaillée. Auparavant, certains termes utilisés dans la définition des composés de formule (I) vont être définis.

Par « alkyle », on entend une chaîne hydrocarbonée saturée qui peut être linéaire ou ramifiée. De préférence, le terme alkyle désigne, à moins qu'il n'en soit spécifié autrement, un groupe alkyle comprenant de 1 à 12 atomes de carbone et préférentiellement de 1 à 6 atomes de carbone, et notamment un groupe (C₁-C₄)alkyle. Méthyle, éthyle, n-propyle, isopropyle, iso-butyle et tert-butyle sont des exemples de groupes (C₁-C₄)alkyle (alkyle avec 1 à 4 atomes de carbone).

Par « aryle, », on entend un cycle mono-, bi- ou polycyclique, hydrocarboné insaturé comportant de préférence de 5 à 20 chainons, préférentiellement de 5 à 15 chainons et comprenant au moins un cycle aromatique alternant liaisons simples et liaisons doubles. A titre d'exemples de groupe aryle, on peut citer les groupes phényle, naphthyle, anthrancényle, phénanthrényle et cinnamyle. Le terme aryle inclut également de tels cycles mono-, bi- ou polycyclique, hydrocarboné insaturé dont l'un des carbones constitutifs se trouve sous la forme carboxy -C(O)- tel que le 1*H*-phénalène-1-one (CAS no. 548-39-0).

Par « glycosidase », on entend une enzyme glycoside hydrolase qui a la capacité de catalyser l'hydrolyse de liaisons glycosidiques, de manière à libérer au moins un composé osidique.

Par groupe « glycosyle », on entend tout sucre mono ou poly saccharide lié au reste de la molécule par un lien glycosyle, c'est-à-dire via son carbone anomérique et donnant lieu à un O/O-acétal, Le carbone anomérique peut adopter la configuration alpha ou beta. A titre d'exemple de groupe R₀, on peut citer les groupements mono-glycosyle, c'est-à-dire formé d'une seule unité saccharidique et poly-glycosyle c'est-à-dire formé de plusieurs unités saccharidiques identiques ou différentes. Les unités saccharidiques peuvent être notamment du type hexoses ou pentoses, et choisis parmi le galactose, le glucose, le mannose, le gulose, l'allose, l'altrose, l'idose, le talose, le fucose, le fructose, l'arabinose, le lyxose, le ribose et le xylose, par exemple. Les unités saccharidiques peuvent être de stéréochimie L ou D. Tous les groupements glycosyle possibles constituant des substrats de glycosidase peuvent être utilisés en tant que R₀. Les unités glycosyle peuvent être fonctionnalisées ou non, notamment avec un groupement acétyl ou amino. Les N-acétylhexosamines sont des exemples de groupe glycosyle. Le plus souvent, le groupe R₀ comprendra de 1 à 50 unités saccharidiques. Dans le cas d'un polyglycosyle, il pourra s'agir d'un homopolymère ou d'un copolymère avec une structure aléatoire, alternée ou block.

Les groupements R₀ ont la caractéristique de pouvoir être clivés du reste de la molécule sous l'action d'une enzyme glycosidase. L'enzyme joue le rôle de catalyseur de la coupure entre R₀ et l'atome d'oxygène auquel il est lié. Une telle coupure peut être la conséquence d'une hydrolyse en milieu aqueux pour laquelle l'enzyme glycosidase va jouer le rôle de catalyseur. C'est pourquoi la dite enzyme glycosidase est dite catalytiquement active. Des exemples de tels groupes R₀ se comportant comme des substrats de glycosidase sont donnés ci-après : les groupements mono-glycosylés choisis parmi le galactosyle, le glucosyle, le mannosyle, le gulosyle, l'allosyle, l'altrosyle, l'idosyle, le talosyle, le fucosyle, le fructosyle, l'arabinosyle, le lyxosyle, le ribosyle, le xylosyle, le glucuronyle et le N-acétyl-hexosaminyle et les groupements polyglycosylés constitués de plusieurs de ces groupements monoglycosylés identique ou différents,

Le groupement R₀ sera, de préférence, choisi de manière à être spécifique d'une glycosidase d'intérêt. En revanche, certaines glycosidase ont la capacité de cliver un ensemble de groupements R₀ différents ; parmi elles, on peut citer la hexosaminidase.

A titre d'exemples d'enzyme glycosidase qui peuvent êtres ciblés par les sondes fluorogèniques selon l'invention, on peut citer la N-acétyt-β-galactosaminidase ; la N-acétyl-p-glucosaminidase ; la α-amylase ; la α-arabinofuranosidase ; la α-arabinosidase ; la β-cellobiosidase ; la β-chitobiosidase ; la α-galactosidase ; la β-galactosidase ; la α-glucosidase ; la β-glucosidase ; la β-glucuronidase ; la α-maltosidase ; la α-mannosidase ; la β-mannosidase ; la β-xylosidase ; la β-D-fucosidase ; la α-L-fucosidase ; la β-L-fucosidase ; la L-iduronidase et la cellulase (Orenga, S., James, A. L., Manafi, M., Perry, J. D., & Pincus, D. H. (2009). Enzymatic substrates in microbiology. Journal of Microbiological Methods, 79(2), 139-155).

La « fluorescence » est la propriété par laquelle une molécule qui est excitée par de la lumière d'une longueur d'onde donnée émet de la lumière à une plus grande longueur d'onde. La fluorescence est un phénomène qui résulte de l'interaction d'un fluorophore avec un photon incident. Ce processus est appelé excitation. L'absorption du photon amène un électron dans le fluorophore à passer de son état fondamental à un niveau d'énergie supérieur. Ensuite, l'électron revient à son niveau original en émettant un photon. Ce processus est appelé émission de fluorescence. Le fluorophore émet ensuite de la lumière à une plus grande longueur d'onde que celle du photon absorbé. Ceci est dû simplement au fait que l'énergie du photon émis est inférieure à celle du photon absorbé, du fait de la dissipation d'énergie pendant la durée de vie de l'état excité. Cette définition est donnée dans la demande de brevet WO 2004/058787.

Les composés (I) selon l'invention sont appelés « substrat de glycosidase » car ils sont transformés en une autre substance pendant une réaction chimique, en particulier une hydrolyse, catalysée par une glycosidase. Pendant une telle réaction, les composés (I) (appelés également « sonde ») sont clivés sous l'action de la glycosidase cible, ce qui conduit à la formation d'un précipité fluorescent et d'un produit non fluorescent.

Le groupe OR₄ est sélectionné de manière à ce que le précipité fluorescent obtenu qui correspond à R₄OH soit un fluorophore ESIPT. lesdits groupes pouvant être substitués ou non substitués,

Les fluorophores ESIPT montrent un déplacement de Stokes qui dépasse les 100 nm et atteint souvent 200 nm. Tous les fluorophores ESIPT perdent cette émission de fluorescence correspondant à un déplacement de Stokes supérieur à 100 nm, si leur groupement OH du type phénolique est alkylé, acylé ou autrement fonctionnalisé, ce qui empêche de transférer un atome d'hydrogène à l'hétéroatome voisin (voir les représentations pages 19 et 21), lors de l'excitation par irradiation, et ainsi empêche rémission de fluorescence caractéristique du processus de transfert de proton.

Les dérivés OR₄ du type phénoxy, correspondent, selon l'invention, aux structures (A) ou (B) suivantes : 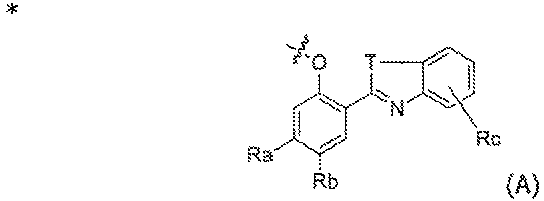 dans laquelle
- T est -NH-C(O)-, -S-, -O-, -NH, N-alkyle ou N-aryle,
- Ra est l'hydrogène ou un substituant carboné attracteur d'électrons choisi parmi -CN ou -COORd, avec Rd qui représente un groupe (C₁-C₄)alkyle, ou bien Ra est -CONReRf, avec Re et Rf, identiques ou différents, qui représentent l'hydrogène ou un groupe (C₁-C₄)alkyle, ou bien Ra est -CF₃ ou un groupe 2-oxazolyle, 2-thiazolyle, 2-imidazolyle, 2-benzoimidazolyle, 4-pyrimidinon-2-yle ou quinazolinon-2-yle,
- Rb est l'hydrogène, un atome de chlore, brome, iode ou fluor, -OH, -NH₂, -NRgRh, -NHRg ou -ORg, avec Rg et Rh qui représentent chacun indépendamment un (C₁-C₄)alkyle,
- ou bien Ra et Rb sont liés entre eux pour former une chaine hydrocarbonée comprenant 4 ou 5 chainons, saturée ou insaturée, non substituée, éventuellement interrompue par un ou plusieurs hétéroatomes choisis parmi N, S et O,
- Rc est l'hydrogène, Br, Cl, I ou F, 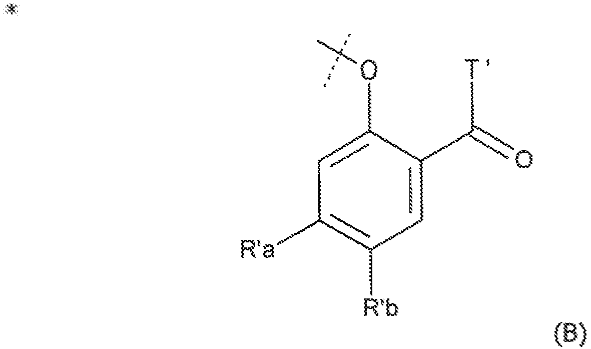
dans laquelle :
- T est NH₂, OH, un groupe aryle, un groupe (C₁-C₄)alkyle, SH, NHR, OR, NRR' ou SR, avec R et R', identiques ou différents, qui représentent un groupe (C₁-C₄)alkyle ou aryle,
- R'a est l'hydrogène ou un substituant carboné attracteur d'électrons choisi parmi -CN, ou -COOR'd, avec R'd qui représente un groupe (C₁-C₄)alkyle, ou R'a est -CONR'eR'f, avec R'e et R'_{f}, identiques ou différents, qui représentent l'hydrogène ou un groupe (C₁-C₄)alkyle, ou bien R'a est -CF₃ ou un 2-oxazolyle, 2-thiazolyle, 2-imidazolyle, 2-benzoimidazolyle, 4-pyrimidinon-2-yle ou quinazolinon-2-yle,
- R'b est l'hydrogène, un atome de chlore, brome, iode ou fluor, -OH, -NH₂, -NR'gR'h ou -OR'g, avec R'g et R'h, identiques ou différents, qui représentent un groupe (C₁-C₄)alkyle
- ou bien R'a et R'b sont liés entre eux pour former une chaîne hydrocarbonée comprenant 4 ou 5 chainons, saturée ou insaturée, non substituée, éventuellement interrompue par un ou plusieurs hétéroatomes choisis parmi N, S et O.

Lorsque la glycosidase cible a libéré un groupe hydroxyle primaire libre à l'extrémité opposée au groupe acyle de l'espaceur -OCH₂C(R₁R₂)N(R₃)C(O)-, ce dernier se cyclise spontanément en libérant ainsi le fluorophore HOR₄.

Dans un mode de réalisation particulier, le substrat de glycosidase selon l'invention est de formule (IA) : 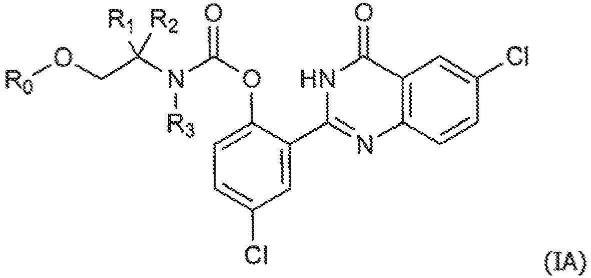 où R₀, R₁, R₂ et R₃ sont tels que définis pour les composés (I).

Dans un mode de réalisation préféré des substrats (I) et (IA) selon l'invention, R₁=H et R₂ et R₃ sont liés entre eux et forment un enchaînement -(CH₂)ₘ- avec m = 3 ou 4. Dans un tel cas, la cinétique d'hydrolyse et de cyclisation, sous l'action d'une enzyme glycosidase est particulièrement satisfaisante.

Cette manière de préorganiser l'espaceur -OCH₂C(R₁R₂)N(R₃)C(O)- pour la cyclisation en un carbamate cyclique, consistant à inclure la liaison entre l'azote du groupement carbamate et son carbone alpha dans un hétérocycle, accélère le processus d'immolation. La liaison entre l'azote du groupement carbamate et son carbone alpha est introduite dans un hétérocycle appartenant à la classe des pyrrolidines (à 5 chaînons) ou à celle des pipéridines (à 6 chaînons), ce qui permet encore d'améliorer la cinétique de transformation catalysée par les glycosidases.

Le **Schéma 4** ci-après montre un exemple de substrat fluorogène (exemple **I.1**) dans le cas où R₂ et R₃ sont liés entre eux et forment un enchaînement -(CH₂)ₘ- avec m = 4 et les cascades de réactions selon lesquelles il se fragmente, après initiation due au clivage par la glycosidase cible.

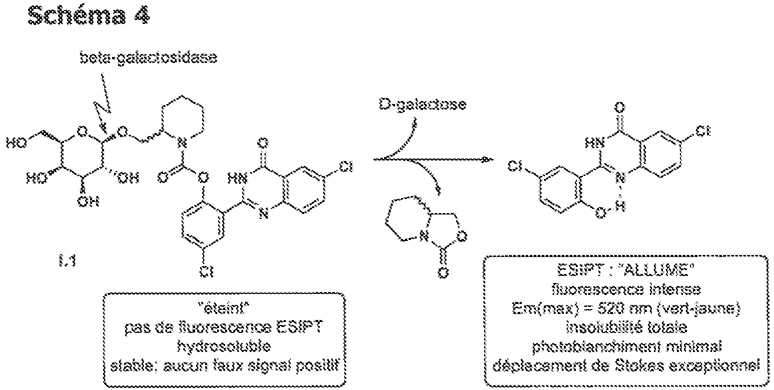

Les substrats **I.1** à **I.4** suivants représentent quatre exemples selon l'invention dans le cas où R₂ et R₃ sont liés entre eux et forment un enchaînement -(CH₂)ₘ- avec m = 3 ou 4.

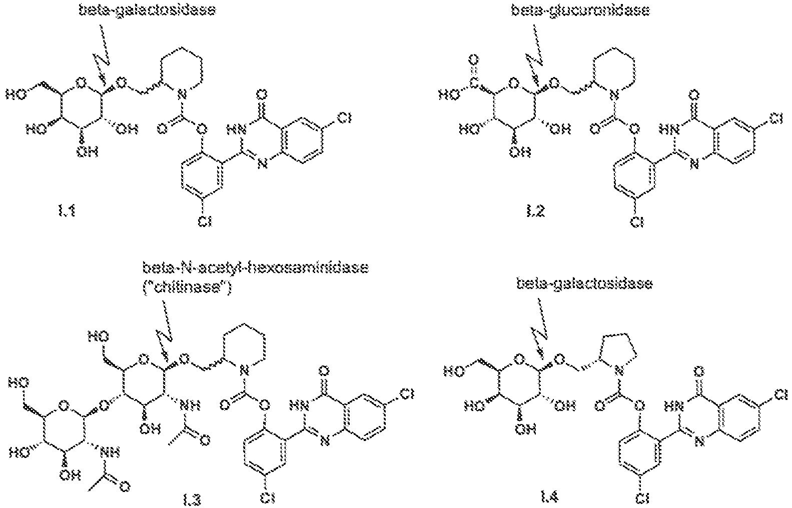

Les exemples 1.1 à 1.4 comprennent le fluorophore ESIPT classique dichloro-HPQ. Cependant, cette invention est applicable à tous les substituants R₄ permettant d'obtenir un phénomène ESIPT, car la chimie de suppression de la fluorescence ESIPT est identique et simple : l'incorporation de l'hydroxyle du fluorophore dans le groupement carbamate empêche la formation de la liaison hydrogène interne. Pour la préparation des dérivés du dicloro-HPQ, on peut se référer à EP 0641 351 ou WO 2004/058787, par exemple.

Certains exemples de fluorophores phénoliques ESIPT qui sont décrits dans la littérature, avec précision de leurs maxima d'excitation et d'émission, et de leur rendement quantique, ("QY") sont énumérés ci-après. 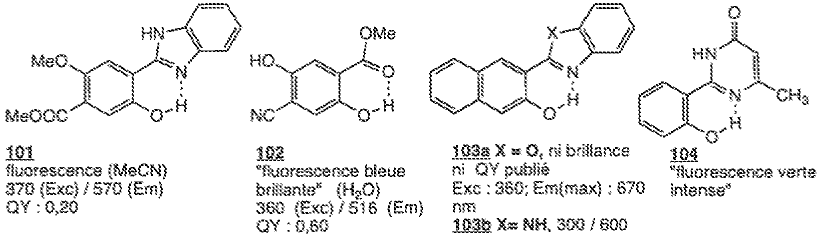 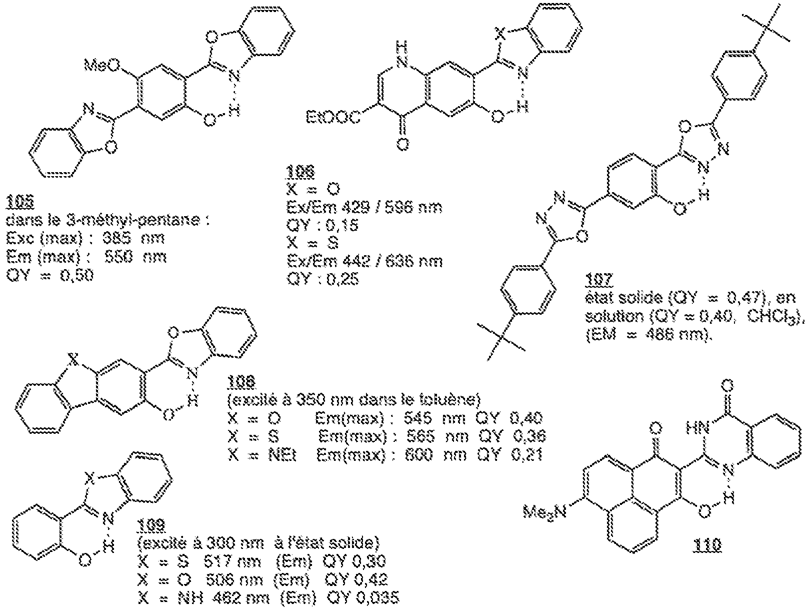
**101 :** Stefani, V., et al. Dyes and Pigments (1992)20, 97-107.
**102 :** Yamada, S., et al. Chemistry Letters (1999), 197-198.
**103a :** Nagaoka, S., et al. Journal of Photochemistry and Photobiology a-Chemistry (1999)122, 151-159.
**103b :** Douhal, A., et al. Chemical Physics Letters (1994)217, 619-625.
**104 :** Kemp, D.S., et al., Journal of Organic Chemistry (1981)46, 1804-1807.
**105** : Mordzinski, A., et al. Journal of Physical Chemistry (1986)90, 1455-1458.
**106** : Lins, G.O.W., et al. Dyes and Pigments (2010)84, 114-120.
**107 :** Seo, J., et al. Journal of Photochemistry and Photobiology a-Chemistry (2007)191, 51-58.
**108 :** Kauffman, J. M., et al. Journal of Heterocyclic Chemistry (1995)32, 1541-1555.
**109 :** Heller, A., et al. J. Phys. Chem. (1970)74, 4473-4480.
**110** : JP2004142131.

Ainsi, le groupe R₄ peut être choisi pour que le substrat de glycosidase libère un fluorophore choisi parmi les fluorophores phénoliques ESIPT **101, 102, 103a, 103b, 104, 105, 106, 107, 108, 109** et **110**, après clivage par la glycosidase cible. D'autres exemples de fluorophores ESIPT pouvant correspondre à HOR₄ sont donnés dans les publications : Prog. Recation Kinetics Vol 19, 1994, 45-91 ; Adv. Mater. 23, 2011, 3615-3642 ;Phys. Chem. Chem. Phys., 2012, 14, 8803-8817, Prog. Reaction Kinetics, Vol. 19, 1994, 211-275, auxquelles on pourra se référer pour plus de détails.

Les substrats (I) selon l'invention peuvent être obtenus par couplage, par exemple avec un composé Cl-C(O)-OR₄ (avec R₄ tel que défini pour les composés de formule (I)), d'une amine de formule (II) : 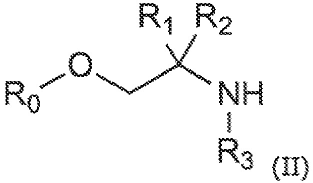 dans laquelle R₀, R₁, R₂ et R₃ sont tels que définis pour les composés de formule (I) ou à partir d'une amine de formule (II') : 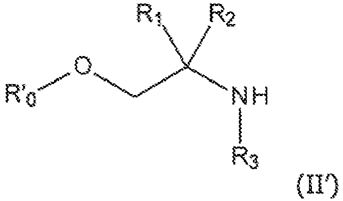 dans laquelle R'₀ est un groupe glycosyle précurseur de R₀ lorsque celui-ci comporte une fonction amide ou acide carboxylique et R₁, R₂ et R₃ sont tels que définis pour les composés de formule (I). En particulier lorsque le groupe glycosyle R₀ comporte une fonction amide ou acide carboxylique, la dite fonction pourra être obtenue à partir respectivement d'une fonction -OH ou -NH₂, selon des techniques bien connues de l'homme de l'art.

Les différents composés selon l'invention peuvent se trouver sous toutes les formes d'isomères optiques possibles, éventuellement en mélange selon toutes proportions, à moins qu'il n'en soit spécifié autrement. Selon un mode de réalisation particulier, les composés selon l'invention comportant un carbone asymétrique se trouvent sous une forme racémique, les formes R et S se trouvant en proportions sensiblement égales. Selon un autre mode de réalisation, les composés de formule (I) de l'invention peuvent se trouver sous une forme enrichie en un diastéréoisomère ou énantiomère, avec un excès diastéréoisomérique ou énantiomérique supérieur à 80 %, voire même supérieure à 95%, voire sous une forme isomérique pure c'est-à-dire avec un excès diastéréoisomérique ou énantiomérique supérieur à 99 %.

Les composés (I) sont isolés sous une forme enrichie en un diastéréoisomère ou énantiomère par les techniques classiques de séparation : on pourra utiliser, par exemple des recristallisations fractionnées d'un sel du racémique avec un acide ou une base optiquement active dont le principe est bien connu ou, le plus souvent, les techniques classiques de chromatographies sur phase chirale ou non chirale.

La présente invention rend l'activité des glycosidases accessible par imagerie par fluorescence utilisant des fluorophores ESIPT, par exemple, du type dichloro-HPQ. Aucun bruit de fond dû à une dégradation spontanée (c'est-à-dire en l'absence de glycosidase cible, en milieu physiologique) n'est observé. La sonde elle-même est peu ou n'est pas fluorescente, en particulier à la longueur d'onde d'émission du fluorophore ESIPT libre sur laquelle l'instrument de détection/imagerie est réglé. La sonde fonctionne ainsi dans le mode éteint/allumé et peut être utilisée pour le développement d'analyses avec une sensibilité maximale. Cette invention permet, en fonction du groupe R₀ sélectionné, de cibler des glycosidases avec une sélectivité élevée pour des groupements glycosyles particuliers. Ceci est possible par couplage d'un tel groupement à l'extrémité de l'espaceur -OCH₂C(R₁R₂)N(R₃)C(O)- intelligent.

Les substrats de la présente invention bénéficient d'une bonne perméabilité vis à vis de la membrane cellulaire par rapport à d'autres substrats d'enzymes fluorogènes connus et seront capables d'entrer aisément dans les cellules (à comparer avec : Duhamel, S. et al. Journal or Microbiological Methods 75 (2008) 269-278), de sorte que ces substrats pourront être utilisés pour différentes applications dans une grande variété de cellules. De plus, les substrats selon la présente invention sont généralement solubles mais très peu fluorescentes sous une forme solubilisée dans l'eau, cependant, ils émettent un signal hautement fluorescent dans une solution aqueuse contenant le substrat et la glycosidase correspondante. Dans le cas de la dichloro-HPQ, ce signal fluorescent est émis à l'état solide par le précipité formé sous l'action de la glycosidase car la dichloro-HPQ est très insoluble dans la plupart des solvants, mais en particulier dans les milieux aqueux. D'autres fluorophores ESIPT ne sont pas non plus particulièrement solubles dans les milieux aqueux, mais la plupart conservent leur capacité à émettre une fluorescence même s'ils précipitent. Les conditions appropriées *in vitro* pour permettre la formation d'un précipité fluorescent lors de l'hydrolyse par une glycosidase sont les milieux purement aqueux, comme un milieu tamponné ou un milieu physiologique. Un point important est que le précipité est formé lors de l'hydrolyse par une glycosidase sans compromettre l'activité glycosidase. Ainsi, leur utilisation pour des études de localisation dans des échantillons biologiques ou la détection de bandes discrètes en PAGE non dénaturante et en transfert de Western est possible. De plus amples détails concernant les conditions et les techniques de détection qui peuvent être utilisées sont donnés dans WO 2004/058787 et EP-0641351 qui peuvent être appliquées directement à la présente invention.

Les sondes selon l'invention sont attractives pour plusieurs applications à haute sensibilité dans les sciences de la vie, et notamment :
(1) le criblage à haut rendement d'une activité glycosidase exprimée par des colonies bactériennes sur une plaque gélosée (analyse sur colonies) ;
(2) la détection *in vitro* de glycosidase dans des liquides biologiques (hématologie et autres) ; (3) la visualisation d'une activité glycosidase au niveau d'une simple cellule en cytométrie de flux; (4) la détection de glycosidases subcellulaires dans des cellules cultivées (microscopie de fluorescence confocale) ; (5) la détection histochimique de glycosidase (à l'échelle tissulaire) ; et finalement (6) l'imagerie *in vivo* d'un animal entier. Les sondes selon l'invention répondent aux exigences des experts dans le domaine de la création de sondes ayant une plus grande robustesse que celles qui sont disponibles actuellement (National Research Council of the U.S.A. (2006) Visualizing Chemistry : The Progress and Promise of Advanced Chemical Imaging, The National Academies Press). En particulier, le domaine à croissance rapide de l'imagerie moléculaire *in vivo* manque de sondes furtives intelligentes qui sont suffisamment robustes, et de complexité moléculaire limitée pour être fabriquées avec des coûts limités (Baker, M. (2010). Whole-animal imaging: The whole picture. Nature 463, 977-980).

Ainsi, les composés de formule (I) et (IA), en tant que substrats de glycosidase selon la présente invention ont un grand nombre d'applications potentielles. Les exemples de telles applications incluent :
(a) la conception d'analyses sur colonies bactériennes. Celles-ci sont actuellement réalisées sur une plaque gélosée (boîte de Petri) où jusqu'à 3 000 colonies peuvent être distinguées sans avoir à les séparer activement dans des compartiments séparés comme les puits contenus dans une plaque à puits multiples. Il est ainsi possible de (1) concevoir des tests sur échantillons cliniques permettant d'identifier parmi un ensemble de lignées bactériennes une lignée pathogène d'intérêt; (2) réaliser des tests parallèles massifs d'une banque de protéines de sa propre production exprimée par un hôte bactérien classique (souvent commercial). Cette collection de protéines peut bien entendu contenir une protéine d'intérêt particulier, par exemple une glycosidase ayant une sélectivité pour un groupement glycosyle spécifique, ou une glycosidase hydrolysant une liaison glycosidique non naturelle. Dans le domaine de l'évolution dirigée des glycosidases en général ou des enzymes en particulier, il existe une forte demande pour des analyses efficaces et sensibles pour cribler de très grands nombres de variants de protéines dépassant aisément 10⁶, L'application de la sonde selon l'invention peut être envisagée le plus aisément par dissolution dans la solution gélosée avant qu'elle soit versée dans la plaque où elle se gélifie. A titre d'alternative, les substrats sont incubés avec les colonies par immersion d'un filtre avant qu'il soit pressé sur les colonies. Le principal avantage auquel la sonde selon l'invention contribue pour une telle analyse sur colonies est la précipitation sur site du fluorophore ; une dilution du signal fluorescent par diffusion est donc très réduite, ce qui permet de plus grandes durées d'incubation et donc une plus grande sensibilité pour l'analyse. Le très grand déplacement de Stokes de la dichloro-HPQ (approximativement 140 nm) ou de tout analogue de la HPQ ne devrait pas rester mésestimé ; il contribue aussi à l'excellente sensibilité et la rend aisément distinguable de la fluorescence native provenant de l'échantillon biologique.
(b) L'imagerie in vitro (histologie) et in vivo. Compte tenu de la très faible solubilité de la dichloro-HPQ libre ou de tout analogue de la HPQ, toute libération de celle-ci dans un environnement biologique complexe permet l'imagerie par fluorescence avec un haut contrôle spatial. L'imagerie par fluorescence est une technique largement appliquée pour mettre en lumière les structures subcellulaires. La localisation d'une activité glycosidase spécifique à haute résolution peut être possible par l'utilisation d'une sonde selon l'invention.

Au contraire, les composés de formule (I) et (IA) qui constituent des sondes fluorescentes passives (celles qui ne sont pas transformées par une enzyme) sont équipées d'un ligand spécifique d'un récepteur cellulaire (ou d'un anticorps spécifique pour un épitope moléculaire de la cellule) pour se fixer sur ce récepteur par des interactions non covalentes ; elles ne sont donc pas dépourvues d'effets de dilution du signal. Par-dessus tout, un récepteur équivaut au mieux à un marqueur fluorescent. La sensibilité d'une analyse basée sur de telles sondes est nécessairement bien plus basse que celle d'une sonde active qui bénéficie de l'amplification catalytique du signal.

Les composés de formule (I) et (IA) selon l'invention peuvent servir aussi pour l'imagerie par fluorescence macroscopique, c'est-à-dire au niveau d'un organisme entier. Si la sonde pénètre dans la paroi cellulaire pour atteindre l'activité Glycosidase d'intérêt, la libération du fluorophore libre exclue la dilution rapide du signal par partage avec l'espace extracellulaire. Généralement, ceci permet des durées d'incubation prolongées avant l'imagerie, qui sont particulièrement utiles quand l'activité enzymatique est exprimée faiblement.

Les substrats de glycosidase selon l'invention peuvent être préparés selon des techniques connues, détaillées dans les exemples suivants. Par exemple, un groupement glycosyle protégé peut être greffé sur le groupe espaceur. Après une étape de couplage pour introduire le fluorophore *via* son hydroxyle phénolique, le groupement glycosyle peut être déprotégé. La sonde complète ainsi obtenue peut être purifiée, avant l'utilisation, par des techniques conventionnelles.

Les exemples suivants illustrent l'invention, mais n'ont aucun caractère limitatif.

### Généralités

La chromatographie sur colonne a été réalisée sur gel de silice 60-mesh (40-63 µm). Les spectres de RMN de ¹H et de ¹³C ont été enregistrés à 500 MHz et à 125 MHz, respectivement, dans le chloroforme deutéré. Les déplacements chimiques (δ) sont indiqués en ppm et notés en référence au tétraméthylsilane ou d'après des signaux résiduels du solvant ; les abréviations s = singulet, d = doublet, t = triplet, m = multiplet, br = large sont utilisées. Les constantes de couplage de RMN (J) sont indiquées en Hertz. Les analyses par fluorescence ont été réalisées dans des plaques en polypropylène noir à 96 (Corning, Corning Inc.) ou à 384 (NUNCLONE, Nunc Inc.) puits, et enregistrées sur un fluorimètre à microplaques (Mithras LB940 de Berthold Technologies). Sauf quand cela est spécifié, les produits chimiques ont été achetés avec la qualité de réactif analytique et utilisés sans autre purification.

Le DCM sec commercial a été séché et purifié par passage sur colonne d'alumine activée sous argon (GT S100 Solvant Station System). La TEA a été distillée à partir de l'hydrure de calcium et conservée sur des pastilles de KOH. Les autres réactifs notés secs ont été séchés sur tamis moléculaires. Si le contraire n'est pas indiqué, toutes les réactions ont été conduites sous atmosphère d'air avec des solvants et réactifs commerciaux, sans séchage ou purification supplémentaire. De l'eau millipore obtenue à partir d'un système de purification Elga Purelab a été utilisée dans toutes les expériences.

Les abréviations suivantes sont utilisées :
TEA = triéthylamine
EA = acétate d'éthyle
Cy = cyclohexane
DCM = dichlorométhane

### Exemple I.1

Le substrat 1.1 est préparé comme décrit sur le **Schéma 5** suivant.

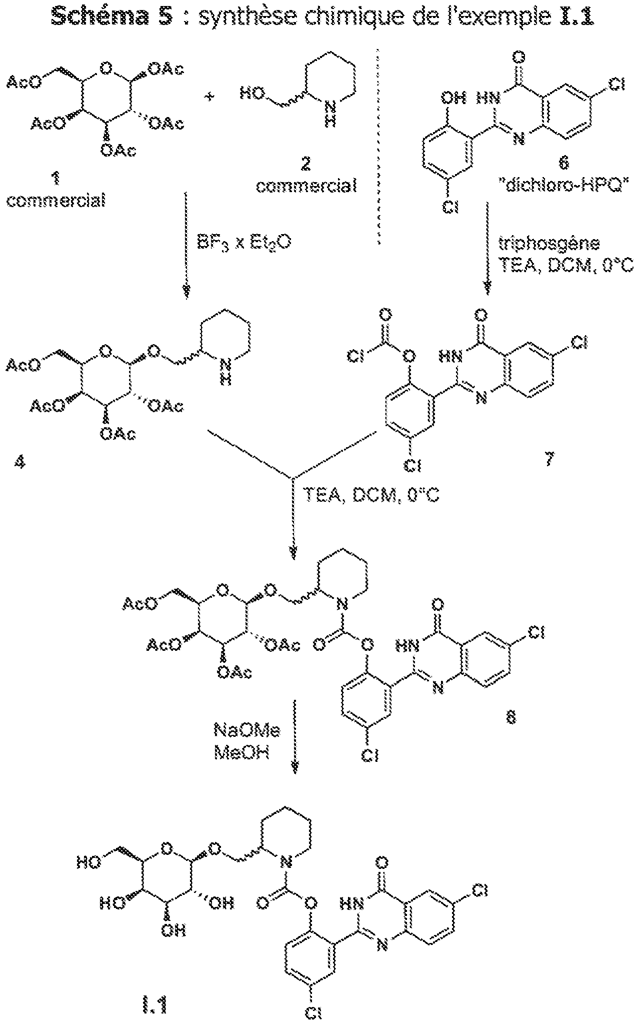

### Préparation du composé 4 :

### (adapté de Li C., Wang W.-T., Tetrahedron Lett. 2002, 43, 3217)

A une solution de pentaacétate de β-D-Galactose **1** (2 g ; 5,1mmol) sous Argon dans le DCM anhydre (14 mL) est ajouté le 2-pipéridinemethanol 2 (887 mg ; 7,7 mmol). Le mélange réactionnel est alors refroidi à 0°C et l'étherate de trifluoroborate (6,3 mL; 48,7 mmol) est additionné goutte à goutte, et ce mélange est agité à cette température pendant une heure puis porté à 35°C pendant 16 heures. Ce mélange réactionnel est partitionné entre le DCM et une solution saturée de carbonate de potassium (Na₂CO₃). La phase organique est lavée deux fois avec la solution saturée de Na₂CO₃ et une fois avec de la saumure saturée. La phase organique est ensuite acidifiée avec une solution aqueuse de KH₂PO₄ (10%) et 1 mL de HCl (1 M) pour obtenir un pH d'environ 3 et est lavée deux fois avec la solution aqueuse de KH₂PO₄ (10%). Les phases aqueuses acides réunies sont lavées une fois avec du DCM puis basifiées avec une solution saturée de Na₂CO₃ et extraites trois fois avec du DCM. Les phases organiques basiques recombinées sont séchées (Na₂SO₄), filtrées et évaporées sous pression réduite pour donner le composé **4** sous la forme d'un solide orange (1,327 g ; 58 %). Les spectres RMN de ce composé sont très complexes mais montrent clairement la présence de deux diastéréoisomères (dus au produit commercial **2** qui est racémique) et que l'on a bien formé la liaison anomérique p (couplage du proton anomérique de 7,9 Hz, caractéristique de ce type de liaison).
¹H NMR (500 MHz, CDCl₃) δ = 5.36 (t, 1H), 5.17 (ddd, 1H), 4.99 (dd, 1H), 4.44 (2xd, *J* = 7.9, 1H), 4.17 - 4.09 (m, 2H), 3.88 (td, 1H), 3.84 (dd, 0,5H), 3.69 (dd, 0,5H), 3.48 (dd, 0,5H), 3.29 (t, 0,5H), 2.79 - 2.66 (m, 1H), 2.62-2.56 (m, 2H), 2.45 (br s, 1H), 2.13 (s, 3H), 2.04 (d, 3H), 2.03 (d, 3H), 1.96 (s, 3H), 1.82 -1.08 (m, 6H).
¹³C NMR (125 MHz, CDCl₃) δ = 170.5, 170.4, 170.3, 170.2, 169.7, 169.6, 101.8, 101.6, 75.3, 75.2, 71.1, 71.0, 70.8, 70.7, 69.1, 67.2, 61.4, 46.6, 28.7, 28.3, 24.4, 24.3, 20.9, 20.8, 20.7, 20.6.
Masse observée : m/z : [M+H]⁺ = 446,2

### Préparation du composé 8 :

### (adapté de Thorn-Seshold O., Vargas-Sanchez M., McKeon S., Hasserodt J., Chem. Comm. 2012, 48, 6253)

Un ballon bicol contenant le composé **6** (313 mg ; 1,1 mmol) subit trois purges vide/argon et du DCM anhydre (3 mL), ainsi que de la TEA sèche (300 µL ; 2,0 mmol) sont introduits. La suspension résultante est refroidie à 0°C et une solution de triphosgène (990 mg ; 3,3 mmol) dans le DCM sec (4 mL) y est additionnée. Le mélange réactionnel résultant est agité à cette température pendant une heure puis les volatiles sont évaporés grâce à une pompe à vide et collectés dans un piège à azote liquide. Alors que le liquide piégé qui contient du phosgène hautement toxique est éliminé en versant délicatement le mélange de volatiles dans une solution d'éthanol dans le DCM (1/1, v/v), le solide vert résultant est placé sous argon et suspendu dans du DCM sec (4 mL), de la TEA sèche (300 µL; 2,0 mmol) est ajoutée et le mélange est refroidi à 0°C avant qu'une solution du composé **4** (470 mg ; 1,00 mmoi) dans le DCM sec (4 mL) soit additionnée goutte à goutte. Le mélange réactionnel résultant est agité à cette température pendant une heure, puis à température ambiante pendant 16 heures avant d'être extrait deux fois avec une solution de Na₂CO₃ saturée. La phase organique contenant une suspension est filtrée sur filtre à plis et extraite une nouvelle fois avec de l'eau désionisée puis avec une solution de saumure saturée. Cette phase organique est séchée (Na₂SO₄), filtrée et évaporée sous pression réduite pour donner un solide beige. Ce brut réactionnel est purifié par deux colonnes de chromatographie sur silice successives avec comme éluant un mélange acétate d'éthyle / éther de pétrole 4/6 puis 5/5 (v/v) pour la première et un gradient des mêmes étuants 1/3, 1/2, 4/6, 45/55, 5/5, 55/45 (v/v) pour donner au final le composé **8** sous la forme d'un solide incolore (332 mg ; 43%) ayant une légère fluorescence bleue lorsqu'il est placé sous une lampe à 365 nm.
¹H NMR (500 MHz, CDCl₃) δ = 10.28 - 10.16 (m, 1H), 8.25 (s, 1H), 8.01-8.00 (m, 1H), 7. 73 (s, 2H), 7.52 (s, 1H), 7.29 - 7.36 (m, 1H), 5.39 (s, 1H), 5.25 - 5.17 (m, 1H), 5.01 (d, 1H), 4.64 - 4.38 (m, contenant le H_{anomérique} avec *J* = 7,9Hz, 2H), 4.20 - 4.01 (m, 4H), 3.99 - 3.90 (m, 1H), 3.60 - 3.56 (m, 1H), 3.09 - 2.76 (m, 1H), 2.16 (s, 3H), 2.05 (s, 3H), 1.98 (s, 6H), 1.76 - 1.65 (m, 6H).
¹³C NMR (125 MHz, CDCl₃) δ = 170.6, 170.3, 160.6, 153.7, 147.6, 153.3, 133.3, 132.6, 130.6, 129.9, 128.1, 126.1, 125.3, 122.5, 101.9, 101.7, 101.3, 101.2, 71.0, 68.9, 68.6, 67.5, 67.2, 51.4, 50.6, 40.1, 25.2, 20.9, 20.8, 20.8,19.4,19.3.
Masse observée : m/z : [M+H]⁺ = 778,0

### Préparation du composé I.1 :

A une solution du composé **8** (150 mg ; 0,17 mmol) dans le méthanol anhydre (3 mL) placée sous argon et refroidie à 0°C est ajoutée goutte à goutte une solution de méthanolate de sodium dans le méthanol (1 M ; 600 µL ; 0,6 mmol). Après une heure de réaction à 0°C, un suivi par masse indique la fin de la réaction et de la résine Dowex® 50X8-100 est additionnée jusqu'à obtenir un pH légèrement acide. Le milieu réactionnel est alors filtré sur fritté puis évaporé sous pression réduite pour donner le composée **I.1** sous la forme une poudre rosée (83 mg ; 88%).
¹H NMR (500 MHz, CD₃OD) δ = 8.23 - 8.21 (m, 1H), 7.89 - 7.72 (m, 3H), 7.63 (d, 1H), 7.39 (t, 1H), 4.22 (br s, 0.5H), 4.19 (d, H_{anomérique} avec *J* = 7.5Hz, 0.4H), 3.98 (d, H_{anomérique} avec *J* = 7.5Hz, 0.6H), 3.88 (t, 0.5H), 3.82 (br s, 2H), 3.76 - 3.68 (m, 3H), 3.62 - 3.57 (m, 2H), 3.51 - 3.38 (m, 3H), 3.38 - 3.35 (m, 1H), 3.24 - 3.17 (m, 0.8H), 2.10 - 1.76 (m, 5H), 1.33 - 1.26 (m, 0.6H).
¹³C NMR (125 MHz, DMSO-*d₆*) δ = 160.7, 151.3, 150.7, 147.9, 147.0, 134.8, 134.7, 131.4, 131.3, 130.0, 129.5, 129.1, 128.3, 128.2, 124.9, 124.8, 122.3, 103.7, 75.2, 75.1, 73.5, 70.4, 68.1, 68.0, 67.7, 60.2, 59.2, 58.8, 56.8, 46.8, 46.7, 30.0, 27.3, 26.9, 22.9, 22.3, 14.7.
HPLC-MS : colonne: Phenomenex Polar-RP
éluant : Acétonitrile / Eau : 35/65
temps de prétention : 9,42 min
masses observées : m/z : [M+H]⁺ = 596,0 , [M+Na]=618,0

### Exemple I.4 :

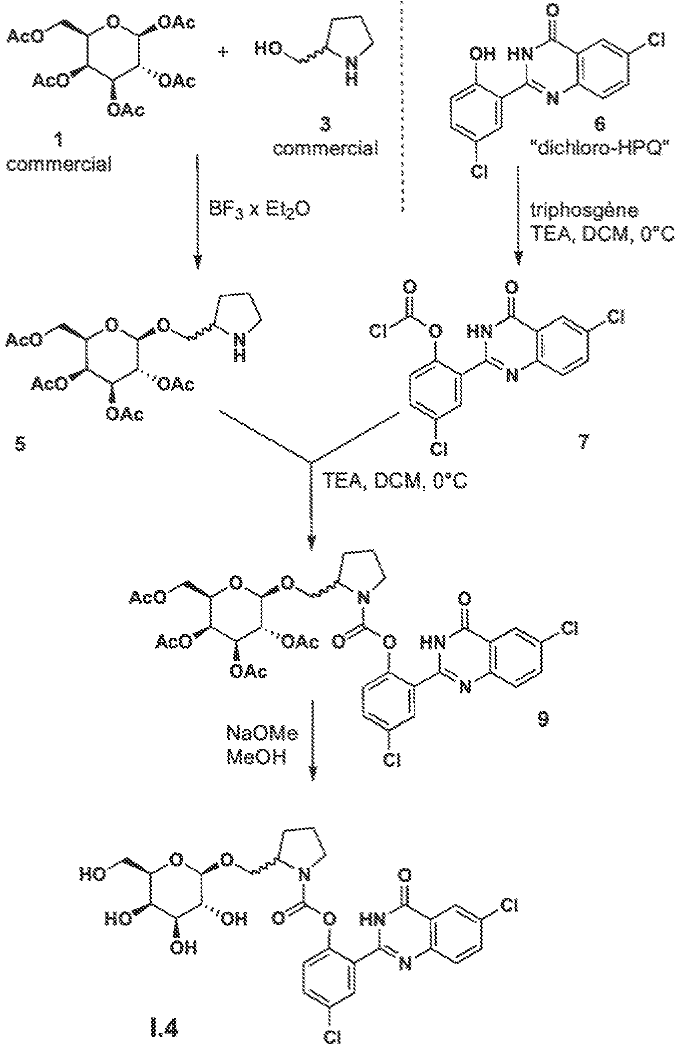

### Préparation du composé 5 :

### (adapté de Li C., Wang W.-T., Tetrahedron Lett. 2002, 43, 3217)

Le composé **5** est préparé de manière similaire au composé **4,** mais en utilisant le 2-pyrrolidinemethanol **3** (787 mg ; 7,7 mmol) comme réactif pour donner le composé **5** sous la forme d'un solide orange (730 mg, 33 %). Les spectres RMN de ce composé sont très complexes mais montrent clairement la présence d'un seul énantiomère (le produit commercial **3** est énantiopure) et que l'on a bien formé la liaison anomérique β (couplage du proton anomérique de 7,9 Hz, caractéristique de ce type de liaison).
¹H NMR (500 MHz, CDCl₃) δ = 5.39 (d, 1H), 5.20 (dd, 1H), 5.02 (dd, 1H), 4.51 (d, *J* = 7.9, 1H), 4.19 - 4.13 (m, 2H), 3.94 - 3.9 (m, 2H), 3.72 (q, 1H), 3.43 (dd, 1H), 3.33 - 3.27 (m, 1H), 3.03 - 2.97 (m, 1H), 2.93-2.87 (m, 1H), 2.15 (s, 3H), 2.07 (s, 3H), 2.06 (s, 3H), 1.98 (s, 3H), 1.85 - 1.73 (m, 4H), 1.45 - 1.35.
¹³C NMR (125 MHz, CDCl₃) δ = 170.8, 170.6, 170.4, 170.3, 170.2, 170.1, 101.4, 71.5, 71.1, 70.7, 68.7, 67.2, 61.5, 61.4, 58.8, 46.1, 26.9, 24.2, 23.1, 21.1, 21.1, 20.9, 20.8, 20.8, 20.7.
Masse observée : m/z : [M+H]⁺ = 432,2

### Préparation du composé 9 :

### (adapté de Thorn-Seshold O., Vargas-Sanchez M., McKeon S., Hasserodt J., Chem. Comm. 2012, 48, 6253)

Le composé **9** est préparé de manière similaire au composé **8,** mais en utilisant le composé **5** (455 mg ; 1,0 mmol) comme réactif pour donner le composé **9** sous la forme d'une poudre blanche (254 mg ; 33 %) ayant une légère fluorescence bleue lorsqu'elle est placée sous une lampe à 365 nm.
¹H NMR (500 MHz, CDCl₃) δ = 10.11 (s, 1H), 8.26 (s, 1H), 8.05 (S, 0.7H), 7. 89 (s, 0.3H), 7.34 (s, 2H), 7.52 (dd, 1H), 7.28 (S, 1H), 5.38 (d, 1H), 5.20 - 5.12 (m, 1H), 5.01 - 4.97 (m, 1H), 4.47 (d, *J =* 7.9, 1H), 4.12 (t, 2H), 4.01 (br s, 1H), 3.89 (t, 1H), 3.82 (t, 1H), 3.70 - 3.64 (m, 2H), 3.61 - 3.55 (m, 1H), 3.52-3.46 (m, 1H), 2.15 (s, 3H), 2.06 - 1.95 (m, 12H).
¹³C NMR (125 MHz, CDCl₃) δ = 170.5, 170.4, 170.2, 169.6, 160.9, 152.4, 149.4, 147.6, 135.4, 133.4, 132.4, 131.9, 130.6, 129.7, 127.5, 126.1, 125.3, 122.4, 101.6, 101.3, 71.0, 70.2, 69.4, 68.9, 68.8, 67.1, 61.3, 61.2, 57.87, 57.2, 47.7, 47.6, 28.8, 27.8, 23.8, 24.0, 20.8.
Masse observée : m/z : [M+H]⁺ = 764,0

### Préparation du composé I.4 :

Le composé **1.4** est préparé de manière similaire au composé **10** mais en utilisant le composé **9** (140 mg ; 0,17 mmol) comme réactif pour donner le composé **1.4** sous la forme d'une poudre rosée (81 mg ; 80 %).
¹H NMR (500 MHz, CD₃OD) δ = 8.23 - 8.21 (m, 1H), 7.89 - 7.72 (m, 3H), 7.63 (d, 1H), 7.39 (t, 1H), 4.22 (br s, 0.5H), 4.19 (d, H_{anomérique} avec *J* = 7.5Hz, 0.4H), 3.98 (d, H_{anomérique} avec *J* = 7.5Hz, 0.6H), 3.88 (t, 0.5H), 3.82 (br s, 2H), 3.76 - 3.68 (m, 3H), 3.62 - 3.57 (m, 2H), 3.51 - 3.38 (m, 3H), 3.38 - 3.35 (m, 1H), 3.24 - 3.17 (m, 0.8H), 2.10 - 1.76 (m, 5H), 1.33 - 1.26 (m, 0.6H).
¹³C NMR (125 MHz, DMSO-*d₆*) δ = 160.7, 151.3, 150.7, 147.9, 147.0, 134.8, 134.7, 131.4, 131.3, 130.0, 129.5, 129.1, 128.3, 128.2, 124.9, 124.8, 122.3, 103.7, 75.2, 75.1, 73.5, 70.4, 68.1, 68.0, 67.7, 60.2, 59.2, 58.8, 56.8, 46.8, 46.7, 30.0, 27.3, 26.9, 22.9, 22.3, 14.7.
HPLC-MS : colonne : Phenomenex Polar-RP
éluant : Acétonitrile / Eau : 35/65
temps de rétention : 9,42 min
masses observées : m/z : [M+H]⁺ = 596,0, [M+Na]=618,0

### Tests enzymatiques

La sonde **I.1** a été évaluée par incubation avec l'enzyme cible, la beta-galactosidase (EC 3.2.1.23 ; « b-gal » ; commercial) dans un milieu *in vitro* dans des micro-plaques à multi-puits conçues pour des lecteurs à fluorescence. La sonde a été évaluée selon les critères suivants :
- Mise en évidence de l'intensité de fluorescence élevée générée par la présence de l'activité enzymatique (« allumé »),
- Mise en évidence de l'absence totale (« éteint ») de fluorescence dans des échantillons qui ne contiennent pas l'enzyme cible (pas de fluorescence intrinsèque),
- Mise en évidence de l'absence de toute dégradation hydrolytique de la sonde au cours du temps démontrant la robustesse de la sonde à pH 7 dans un milieu aqueux (pas de signal faussement positif),
- Mise en évidence de la rapidité de réponse à la présence de l'activité enzymatique permettant d'atteindre rapidement un signal maximal,
- Mise en évidence de la corrélation du signal mesuré avec la concentration de la sonde,
- Mise en évidence de la forte photo-stabilité du fluorophore solide généré sous irradiation prolongée par le lecteur de fluorescence.

Les principaux tests ont été conduits à 25°C, dans des solutions tamponnées à pH 7,4 (pH physiologique). Des tests à 37°C auraient nécessité la protection des puits contre l'évaporation prématurée, par exemple avec la couverture par un film ce qui aurait réduit fortement la sensibilité de la mesure de fluorescence. Une température de 25°C a donc a été préférée.

**Préambule :** Dans le cadre de l'invention, lors de la mise en oeuvre des tests enzymatiques, une fois la concentration minimale permettant de déclencher la nucléation de cristaux atteinte, un fluorophore solide se dépose dans un puits d'une plaque à multi-puits. Le délai associé avec le déclenchement de la nucléation (≥ 10 min) se voit bien sur les courbes de cinétiques dans les figures présentées.

Les tests enzymatiques de la sonde 1.1 qui suivent ont été effectués dans une microplaque à 96 puits dans un spectrofluorimètre à microplaques du type Mithras LB 940 de BERTHOLD TECHNOLOGIES. La longueur d'onde d'excitation a été fixée à 355 nm et celle de la détection à 510nm, conformément aux propriétés spectrales du dichloro-HPQ.

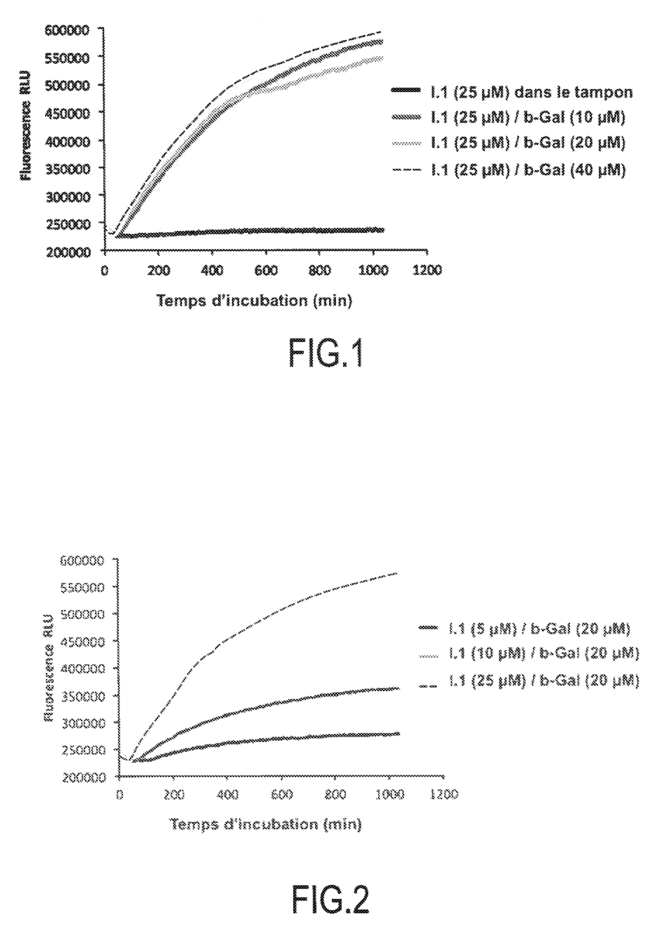

Les résultats obtenus sont présentés sur les **Figures 1** à **4** annexée.
La **Figure** 1 montre l'évolution des signaux de fluorescence (RLU = random luminescence units) causée par la mise en contact du composé **I.1** à une concentration fixe (25 µM) avec respectivement trois concentrations différentes de l'enzyme b-gaL Un 4^{ème} tracé correspond au contrôle négatif, i.e. à l'évolution du signal de fluorescence du composé **I.1** en absence d'enzyme. Cette courbe reste plane et démontre ainsi l'absence totale d'un signal de fluorescence en absence du stimulus biochimique : le signal de fond n'est donc en rien modifié par la présence de la sonde intacte, On constate également une indépendance du signal par rapport à la concentration en enzyme cible dans l'intervalle de concentration choisi.
La **Figure 2** montre l'évolution des signaux de fluorescence (RLU = random luminescence units) causée par la mise en contact de l'enzyme cible b-gal à une concentration fixe (20 µM) avec respectivement trois concentrations différentes de la sonde **I.1.** On constate une forte croissance du signal avec l'augmentation de la concentration en sonde.

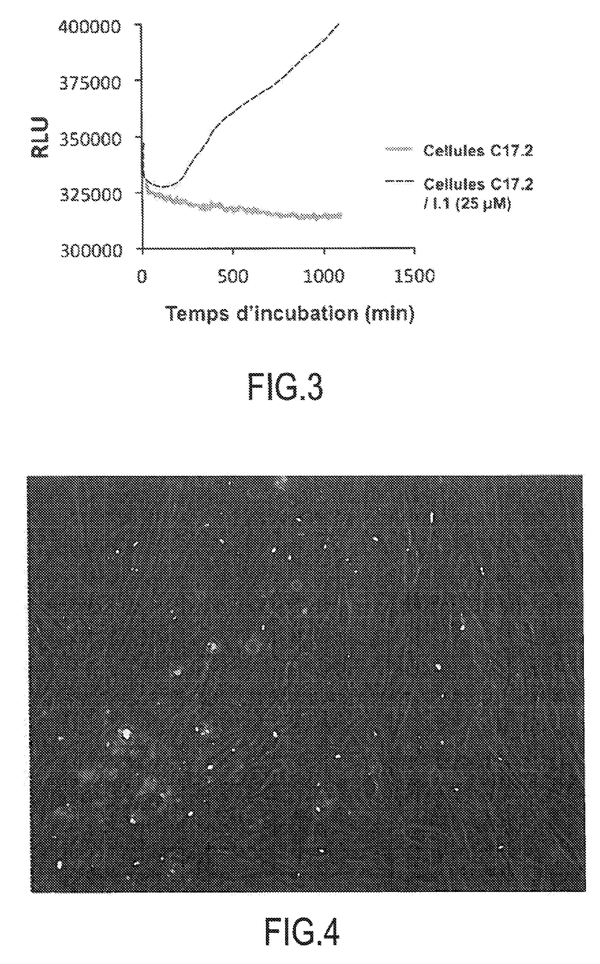

La **Figure 3** montre l'ncubation de la sonde **1.1** (25 µM) avec des cellules de type C17.2 qui expriment l'enzyme cible b-gal de façon constitutive. L'expérience a été menée dans une plaque à culture cellulaire en absence de sérum, et le signal de fluorescence a été mesuré par le spectrofluorimètre à microplaques du type Mithras LB 940 de BERTHOLD TECHNOLOGIES. La courbe correspondant au contrôle négatif démontre l'absence d'une quelconque génération de signal fluorescent. La **Figure 3** met en évidence le pouvoir de la sonde **I.1** de franchir la membrane cellulaire, de rentrer en contact avec l'enzyme cible b-gal qui se trouve au sein du cytoplasme, et de se faire transformer par l'enzyme.
La **Figure 4** montre une image de microscopie de fluorescence de cellules qui expriment de façon constitutive la b-gal et qui ont été incubées par la sonde I.1 à 25 µM pendant 48 heures dans un milieu sans sérum. On peut clairement identifier des cellules ou la concentration en **I.1** transformée a atteint un niveau qui laisse précipiter le dichloro-HPQ fortement fluorescent (taches blanches).

## Revendications

1. Composés de formule (I) : 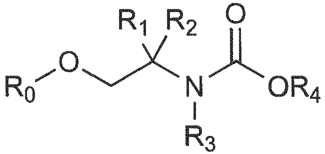 dans laquelle :
- R₀ est un groupement glycosyle lié par son carbone anomérique au reste de la molécule, la liaison R₀-O étant susceptible d'être clivée, par hydrolyse, en présence d'une enzyme glycosidase agissant en tant que catalyseur de la réaction de clivage,
- R₁ est un (C₁-C₄)alkyle ou un atome d'hydrogène et R₂ et R₃ sont liés entre eux et forment avec les atomes de carbone et d'azote auxquels ils sont liés un hétérocycle aliphatique choisi parmi la pyrrolidine et la pipéridine,
- R₄ est tel que lorsque la liaison C(O)-OR₄ est clivée, il est libéré sous la forme d'un fluorophore ESIPT HOR₄, avec OR₄ qui est choisi parmi : 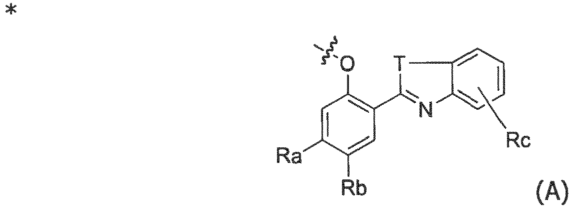 dans laquelle :
- -T- est -NH-C(O)-, -S-, -O-, -NH-, -N(alkyle)- ou -N(aryle)-,
- Ra est l'hydrogène ou un substituant carboné attracteur d'électrons choisi parmi -CN ou -COORd, avec Rd qui représente un groupe (C₁-C₄)alkyle, ou bien Ra est -CONReRf, avec Re et Rf, identiques ou différents, qui représentent l'hydrogène ou un groupe (C₁-C₄)alkyle, ou bien Ra est -CF₃ ou un 2-oxazolyle, 2-thiazolyle, 2-imidazolyle, 2-benzoimidazolyle, 4-pyrimidinon-2-yle ou quinazolinon-2-yle,
- Rb est l'hydrogène, un atome de chlore, brome, iode ou fluor, -OH, -NH₂, -NRgRh, -NHRg ou -ORg, Rg et Rh qui représentent chacun indépendamment un groupe (C₁-C₄)alkyle,
- ou bien Ra et Rb sont liés entre eux pour former une chaine hydrocarbonée comprenant 4 ou 5 chainons, saturée ou insaturée, non substituée, éventuellement interrompue par un ou plusieurs hétéroatomes choisis parmi N, S et O,
- Rc est l'hydrogène, Br, Cl, I ou F, 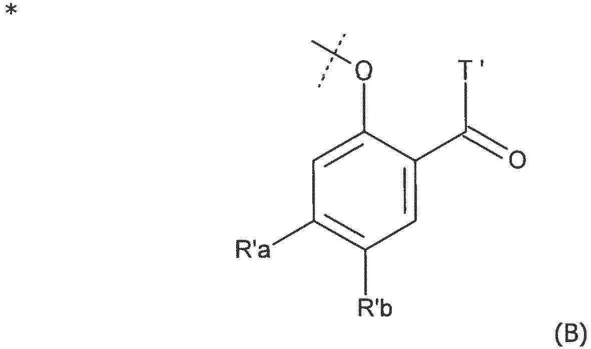
dans laquelle :
- T' est -NH₂, -OH, un aryle, un (C₁-C₄)alkyle, -SH, -NHR, -OR, -NRR' ou -SR, avec R et R', identiques ou différents, qui représentent un (C₁-C₄)alkyle ou un aryle,
- R'a est l'hydrogène ou un substituant carboné attracteur d'électrons choisi parmi -CN, ou -COOR'd, avec R'd qui représente un groupe (C₁-C₄)alkyle, ou R'a est -CONR'eR'f, avec R'e et R'f, identiques ou différents, qui représentent l'hydrogène ou un groupe (C₁-C₄-)alkyle, ou bien R'a est -CF₃, ou un 2-oxazolyle, 2-thiazolyle, 2-imidazolyle, 2-benzoimidazolyle, 4-pyrimidinon-2-yle, ou quinazolinon-2-yle,
- R'b est l'hydrogène, un atome de chlore, brome, iode ou fluor, -OH, -NH₂, -NR'gR'h ou -OR'g, avec R'g et R'h, identiques ou différents, qui représentent un groupe (C₁-C₄)alkyle,
- ou bien R'a et R'b sont liés entre eux pour former une chaine hydrocarbonée comprenant 4 ou 5 chaînons, saturée ou insaturée, non substituée, éventuellement interrompue par un ou plusieurs hétéroatomes choisis parmi N, S et O ;
sous la forme d'un mélange d'isomères optiques selon toutes proportions, ou sous une forme enrichie en un isomère optique.

2. Composés selon la revendication 1 **caractérisés en ce que** R₁=H et R₂ et R₃ sont liés entre eux et forment un enchaînement -(CH₂)ₘ- avec m = 3 ou 4.

3. Composés selon l'une quelconque des revendications précédentes de formule (IA) : 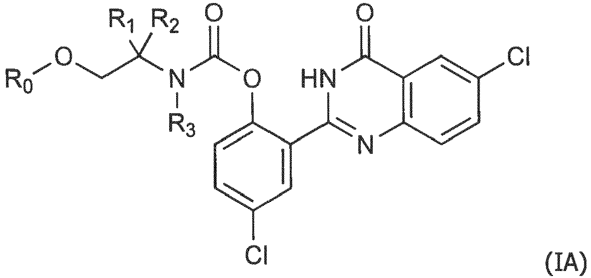 où R₀, R₁, R₂ et R₃ sont tels que définis aux revendications 1 ou 2, sous la forme d'un mélange d'isomères optiques selon toutes proportions, ou sous une forme enrichie en un isomère optique.

4. Composés selon l'une quelconque des revendications précédentes **caractérisés en ce que** R₀ représente un groupement mono-glycosyle ou poly-glycosyle.

5. Composés selon l'une quelconque des revendications précédentes **caractérisés en ce que** R₀ représente un groupement mono-glycosylé choisi parmi le galactosyle, le glucosyle, le mannosyle, le gulosyle, l'allosyle, l'altrosyle, l'idosyle, le talosyle, le fucosyle, le fructosyle, l'arabinosyle, le lyxosyle, le ribosyle, le xylosyle, le glucuronyle et le N-acétyl-hexosaminyle ou un groupement polyglycosylé constitué de plusieurs de ces groupements monoglycosylés identiques ou différents.

6. Composés selon l'une quelconque des revendications précédentes **caractérisés en ce que** le groupement glycosyle R₀ peut être clivé du reste de la molécule par action d'une enzyme glycosidase choisie parmi : la N-acétyl-β-galactosaminidase, N-acétyl-β-glucosaminidase, α-amylase, α-arabinofuranosidase, α-arabinosidase, β-cellobiosidase, β-chitobiosidase, α-galactosidase, β-galactosidase, α-glucosidase, β-glucosidase, β-glucuronidase, α-maltosidase, α-mannosidase, β-mannosidase, β-xylosidase, β-D-fucosidase, α-L-fucosidase, β-L-fucosidase, L-iduronidase et la cellulase.

7. Composés selon l'une quelconque des revendications précédentes pour la détection *in vivo,* chez l'homme ou l'animal, d'une enzyme glycosidase.

8. Procédé pour détecter *in vitro* ou *ex vivo* la présence d'une glycosidase catalytiquement active comprenant les étapes de :
- mise en contact d'un échantillon suspecté de contenir ladite glycosidase avec un composé selon l'une quelconque des revendications 1 à 6 ;
- application de conditions appropriées pour permettre la formation d'un précipité fluorescent par clivage de la liaison covalente entre O et R₀, suivi d'un clivage de la liaison -C(O)-OR₄ suite à une cyclisation de l'espaceur -OCH₂C(R₁R₂)N(R₃)C(O)-,
- analyse quantitative ou qualitative dudit précipité fluorescent.

9. Procédé selon la revendication 8 **caractérisé en ce que** l'analyse du précipité fluorescent comprend:
- une étape d'exposition du précipité fluorescent à une source lumineuse capable de produire de la lumière à une longueur d'onde d'absorption du précipité fluorescent ; et
- une étape de détection de la fluorescence du précipité résultante.

## Patentansprüche

1. Verbindungen der Formel (I): 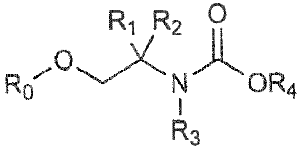 worin:
- Ro eine Glycosylgruppe ist, die durch ihren anomeren Kohlenstoff an den Rest des Moleküls gebunden ist, wobei die R₀-O-Bindung geeignet ist, durch Hydrolyse, in Gegenwart eines Glycosidase-Enzyms, welches als Katalysator der Spaltreaktion wirkt, abgespalten zu werden,
- R₁ ein (C₁-C₄)-Alkyl oder ein Wasserstoffatom ist und R₂ und R₃ untereinander verbunden sind und mit den Kohlenstoff- und Stickstoffatomen, an die sie gebunden sind, einen aliphatischen Heterozyklus, ausgewählt aus Pyrrolidin und Piperidin, bilden,
- R₄ derart ist, dass wenn die C(O)-OR₄-Bindung abgespalten wird, es in Form eines ESIPT-Fluorophors HOR₄ freigesetzt wird, wobei OR₄ ausgewählt ist aus: 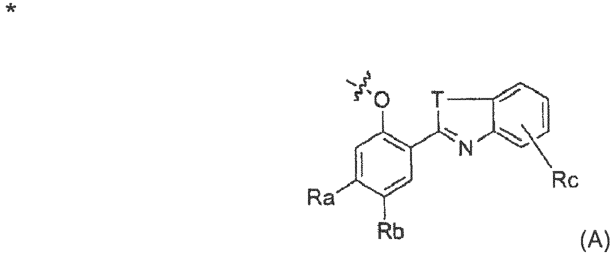
worin:
- -T- -NH-C(O)-, -S-, -O-, -NH-, -N(Alkyl)- oder -N(Aryl)- ist,
- Ra Wasserstoff oder ein Elektronen anziehender Kohlenstoffsubstituent ist, ausgewählt aus -CN oder-COORd, wobei Rd eine (C₁-C₄)-Alkylgruppe darstellt, oder aber Ra -CONReRf ist, wobei Re und Rf, die identisch oder unterschiedlich sind, Wasserstoff oder eine (C₁-C₄)-Alkylgruppe darstellen, oder aber Ra -CF₃ oder ein 2-Oxazolyle, 2-Thiazolyl, 2-Imidazolyl, 2-Benzoimidazolyl, 4-Pyrimidinon-2-yl oder Chinazolinon-2-yl ist,
- Rb Wasserstoff, ein Chlor-, Brom-, Jod- oder Fluoratom, -OH, -NH₂, -NRgRh,-NHRg oder -ORg ist, wobei Rg und Rh jeweils unabhängig eine (C₁-C₄)-Alkylgruppe darstellen,
- oder aber Ra und Rb untereinander verbunden sind, um eine 4- oder 5-gliedrige Kohlenwasserstoffkette, die gesättigt oder ungesättigt, unsubstituiert,
eventuell durch ein oder mehrere Heteroatome, ausgewählt aus N, S und O, unterbrochen ist, zu bilden,
- Rc Wasserstoff, Br, Cl, I oder F ist, 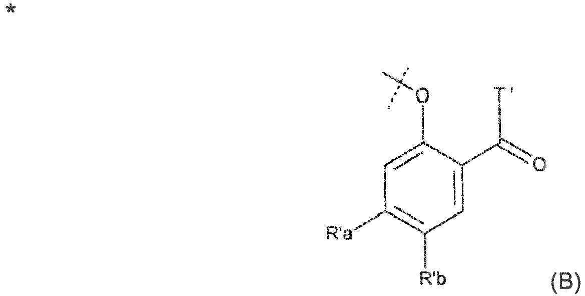
worin:
- T' -NH₂, -OH, ein Aryl, ein (C₁-C₄)-Alkyl, -SH, -NHR, -OR, -NRR' oder -SR ist, wobei R und R', die identisch oder unterschiedlich sind, ein (C₁-C₄)-Alkyl oder ein Aryl darstellen,
- R'a Wasserstoff oder ein Elektronen anziehender Kohlenstoffsubstituent ist, ausgewählt aus -CN, oder -COOR'd, wobei R'd eine (C₁-C₄)-Alkylgruppe darstellt, oder R'a -CONR'eR'f ist, wobei R'e und R'f, die identisch oder unterschiedlich sind, Wasserstoff oder eine (C₁-C₄)-Alkylgruppe darstellen, oder aber R'a -CF₃ ist, oder ein 2-Oxazolyle, 2-Thiazolyl, 2-Imidazolyl, 2-Benzoimidazolyl, 4-Pyrimidinon-2-yl oder Chinazolinon-2-yl,
- R'b Wasserstoff, ein Chlor-, Brom-, Jod- oder Fluoratom, -OH, -NH₂, -NR'gR'h oder-OR'g ist, wobei R'g und R'h, die identisch oder unterschiedlich sind, eine (C₁-C₄)-Alkylgruppe darstellen,
- oder aber R'a und R'b untereinander verbunden sind, um eine 4- oder 5-gliedrige Kohlenwasserstoffkette, die gesättigt oder ungesättigt, unsubstituiert,
eventuell durch ein oder mehrere Heteroatome, ausgewählt aus N, S und O, unterbrochen ist, zu bilden,
in Form einer Mischung aus optischen Isomeren in jedweden Anteilen oder in einer mit einem optischen Isomer angereicherten Form.

2. Verbindungen nach Anspruch 1, **dadurch gekennzeichnet, dass** R₁=H und R₂ und R₃ untereinander verbunden sind und eine -(CH₂)ₘ₋-Verkettung, mit m = 3 oder 4, bilden.

3. Verbindungen nach einem der vorhergehenden Ansprüche, der Formel (IA): 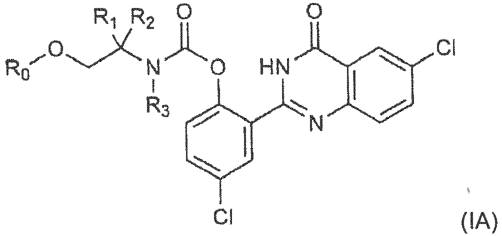 worin R₀, R₁, R₂ und R₃ derart sind, wie sie in den Ansprüchen 1 oder 2 definiert sind, in Form einer Mischung aus optischen Isomeren in jedweden Anteilen oder in einer mit einem optischen Isomer angereicherten Form.

4. Verbindungen nach einem der vorhergehenden Ansprüche, **dadurch gekennzeichnet, dass** R₀ eine Monoglycosyl- oder Polyglycosyl-Gruppe darstellt.

5. Verbindungen nach einem der vorhergehenden Ansprüche, **dadurch gekennzeichnet, dass** R₀ eine Monoglycosyl-Gruppe, ausgewählt aus Galactosyl, Glucosyl, Mannosyl, Gulosyl, Allosyl, Altrosyl, Idosyl, Talosyl, Fucosyl, Fructosyl, Arabinosyl, Lyxosyl, Ribosyl, Xylosyl, Glucuronyl und N-Acetyl-hexosaminyl, oder eine Polyglycosyl-Gruppe, bestehend aus mehreren dieser Monoglycosyl-Gruppen, identisch oder unterschiedlich, darstellt.

6. Verbindungen nach einem der vorhergehenden Ansprüche, **dadurch gekennzeichnet, dass** die Glycosylgruppe R₀ von dem Rest des Moleküls durch Wirkung eines Glycosidase-Enzyms, ausgewählt aus N-Acetyl-β-galactosaminidase, N-Acetyl-β-glucosaminidase, α-Amylase, α-Arabinofuranosidase, α-Arabinosidase, β-Cellobiosidase, β-Chitobiosidase, α-Galactosidase, β-Galactosidase, α-Glucosidase, β-Glucosidase, β-Glucuronidase, α-Maltosidase, α-Mannosidase, β-Mannosidase, β-Xylosidase, β-D-Fucosidase, α-L-Fucosidase, β-L-Fucosidase, L-Iduronidase und Cellulase, abgespalten werden kann.

7. Verbindungen nach einem der vorhergehenden Ansprüche, für den *in vivo-*Nachweis eines Glycosidase-Enzyms bei Mensch oder Tier.

8. Verfahren, um *in vitro* oder ex *vitro* das Vorliegen einer katalytisch aktiven Glycosidase nachzuweisen, welches die Schritte umfasst:
- des Inkontaktbringens einer Probe, die im Verdacht steht, die Glycosidase zu enthalten, mit einer Verbindung nach einem der Ansprüche 1 bis 6,
- des Anwendens von geeigneten Bedingungen zum Ermöglichen der Bildung eines fluoreszierenden Niederschlags durch Spalten der kovalenten Bindung zwischen O und R₀, gefolgt von einer Spaltung der Bindung -C(O)-OR₄ im Anschluss an eine Zyklisierung des Spacers -OCH₂C(R₁R₂)N(R₃)C(O)-,
- des quantitativen oder qualitativen Analysierens des fluoreszierenden Niederschlags.

9. Verfahren nach Anspruch 8, **dadurch gekennzeichnet, dass** die Analyse des fluoreszierenden Niederschlags umfasst:
- einen Schritt, um den fluoreszierenden Niederschlag einer Lichtquelle auszusetzen, die in der Lage ist, Licht mit einer Absorptionswellenlänge des fluoreszierenden Niederschlags zu erzeugen, und
- einen Schritt zum Nachweisen der Fluoreszenz des resultierenden Niederschlags.

## Claims

1. Compounds of formula (I): 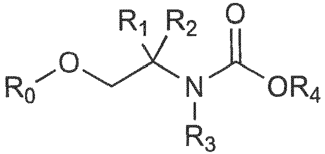 where:
- R₀ is a glycosyl group bonded via its anomeric carbon to the remainder of the molecule, the R₀-O bond being able to be cleaved, via hydrolysis, in the presence of a glycosidase enzyme acting as catalyst of the cleavage reaction;
- R₁ is a (C₁-C₄)alkyl or hydrogen atom and R₂ and R₃ are linked and, together with the carbon and nitrogen atoms to which they are bonded, form an aliphatic heterocycle chosen among the piperine and the pyrrolidine;
- R₄ is such that, when the bond C(O)-R₄ is cleaved, it is released in the form of a fluorophore ESIPT HOR₄, with OR₄ which is chosen among: 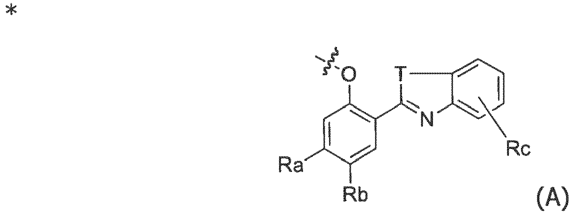 where:
- -T- is -NH-C(O)-, -S-, -O-, -NH-, -N(alkyl)- or -N(aryl)-,
- Ra is hydrogen or an electron-attractor carbon substituent chosen among -CN or -COORd, with Rd representing a (C₁-C₄)alkyl group, or else Ra is -CONReRf with Re and Rf, the same or different, representing hydrogen or a (C₁-C₄)alkyl group, or else Ra is -CF₃ or 2-oxazolyl, 2-thiazolyl, 2-imidazolyl, 2-benzoimidazolyl, 4-pyrimidinon-2-yl or quinazolinon-2-yl;
- Rb is hydrogen, a chlorine, bromine, iodine or fluorine atom, -OH,-NH₂, -NRgRh, -NHRg or -ORg, with Rg and Rh each independently representing a (C₁-C₄)alkyl group;
- or else Ra and Rb are linked together to form a hydrocarbon chain having 4 or 5 members, saturated or unsaturated, unsubstituted, optionally interrupted by one or more heteroatoms selected from among N, S and O;
- Rc is hydrogen, Br, Cl, I or F; 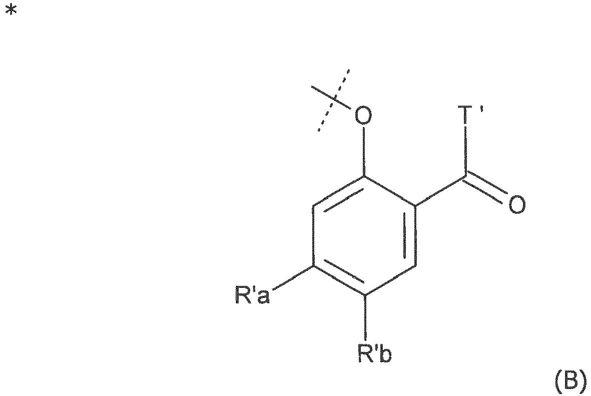
where:
- T' is -NH₂, -OH, an aryl, (C₁-C₄)alkyl, -SH, -NHR, -OR, -NRR' or -SR, with R and R', the same or different, representing a (C₁-C₄)alkyl or aryl,
- R'a is hydrogen or an electron-attractor carbon substituent chosen among -CN, or -COOR'd with R'd representing a (C₁-C₄)alkyl group, or R'a is -CONR'eR'f with R'e and R'f, the same or different, representing hydrogen or (C₁-C₄-)alkyl group, or else R'a is -CF₃, or 2-oxazolyl, 2-thiazolyl, 2-imidazolyl, 2-benzoimidazolyl, 4-pyrimidinon-2-yl, or quinazolinon-2-yl;
- R'b is hydrogen, a chlorine, bromine, iodine or fluorine atom, -OH,-NH₂, -NR'gR'h or -OR'g, with R'g and R'h the same or different representing a (C₁-C₄)alkyl group;
- or else R'a and R'b are linked together to form a hydrocarbon chain having 4 or 5 members, saturated or unsaturated, unsubstituted, optionally interrupted by one or more heteroatoms selected from among N, S and O;
in the form of a mixture of optical isomers in any proportion or in a form enriched with an optical isomer.

2. The compounds according to claim 1 **characterized in that** R₁=H and R₂ and R₃ are linked together and form a-(CH₂)ₘ- chain where m = 3 or 4.

3. The compounds according to any of the preceding claims of formula (IA): 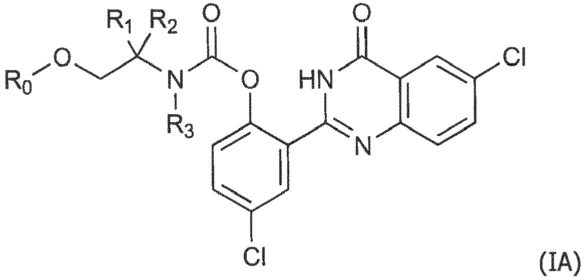 where R₀, R₁, R₂ and R₃ are such as defined in claims 1 or 2, in the form of a mixture of optical isomers in any proportion, or in a form enriched with an optical isomer.

4. The compounds according to any of the preceding claims **characterized in that** R₀ is a mono-glycosyl or poly-glycosyl group.

5. The compounds according to any of the preceding claims **characterized in that** R₁ is a mono-glycosyl group selected from among galactosyl, glucosyl, mannosyl, gulosyl, allosyl, altrosyl, idosyl, talosyl, fucosyl, fructosyl, arabinosyl, lyxosyl, ribosyl, xylosyl, glucuronyl and N-acetyl-hexosaminyl, or a polyglycosyl group formed of several of these monoglycosyl groups, the same or different.

6. The compounds according to any of the preceding claims **characterized in that** the glycosyl group R₀ can be cleaved from the remainder of the molecule by action of a glycosidase enzyme selected from among: N-acetyl-β-galactosaminidase, N-acetyl-β-glucosaminidase, α-amylase, α-arabinofuranosidase, α-arabinosidase, β-cellobiosidase, β-chitobiosidase, α-galactosidase, β-galactosidase, α-glucosidase, p-glucosidase, β-glucuronidase, α-maltosidase, α-mannosidase, p-mannosidase, β-xylosidase, β-D-fucosidase, α-L-fucosidase, β-L-fucosidase, L-iduronidase and cellulase.

7. The compounds according to any of the preceding claims for *in vivo* detection in man or animal of a glycosidase enzyme.

8. A method for the *in vitro* or *ex vivo* detection of the presence of a catalytically active glycosidase, comprising the steps of:
- contacting a sample suspected of contained the said glycosidase with a compound according to any of claims 1 to 6;
- applying suitable conditions to allow the formation of a fluorescent precipitate by cleavage of the covalent bond between O and R₀, followed by cleavage of the -C(O)-OR₄ bond further to cyclisation of the spacer -OCH₂C(R₁R₂)N(R₃)C(O)-,
- quantitative or qualitative analysis of the said fluorescent precipitate.

9. The method according to claim 8 **characterized in that** the analysis of the fluorescent precipitate comprises:
- a step to expose the fluorescent precipitate to a light source capable of producing light at an absorption wavelength of the fluorescent precipitate; and
- a step to detect the fluorescence of the resulting precipitate.
